# EUROPEAN PATENT APPLICATION

(11) **EP 4 141 097 A1**
(43) Date of publication of application: **01.03.2023**
(21) Application number: 20931791.6
(22) Date of filing: 23.04.2020
(51) Int. Cl.: C12M 1/18, C12M 1/16, C12M 1/00

(54) **SEQUENCING CHIP AND PREPARATION METHOD THEREFOR**

(71) Applicant: BGI Shenzhen, Shenzhen, Guangdong 518083 (CN)
(72) Inventor: WANG, Zhaohui, Shenzhen, Guangdong 518083 (CN); LI, Handong, Shenzhen, Guangdong 518083 (CN); LI, Yuan, Shenzhen, Guangdong 518083 (CN); ZHANG, Wenwei, Shenzhen, Guangdong 518083 (CN); CHEN, Ao, Shenzhen, Guangdong 518083 (CN)
(74) Representative: DehnsGermany Partnerschaft von Patentanwälten
(86) International application number: PCT/CN2020/086477
(87) International publication number: WO 2021/212429

(57) **Abstract**

Provided in the present disclosure is a sequencing chip. The sequencing chip includes: a chip main body, nucleic acids, and a phosphonic acid polymer film. The chip main body includes a plurality of chip particles arranged in a same layer, the chip particles are obtained by cutting a chip matrix along cutting lines of a wafer layer, and the chip matrix includes: the wafer layer having the cutting lines uniformly distributed thereon; a first silicon oxide layer made of silicon oxide and formed on an upper surface of the wafer layer; and a transition metal oxide layer made of a transition metal oxide and formed on an upper surface of the first silicon oxide layer. The nucleic acids are fixed on the transition metal oxide layer; and the phosphonic acid polymer film is made of a polyphosphonic acid polymer and formed on an upper surface of the transition metal oxide layer.

## Description

### PRIORITY INFORMATION

None

### TECHNICAL FIELD

The present disclosure relates to the field of biotechnology. Specifically, the present disclosure relates to a sequencing chip and a preparation method therefor.

### BACKGROUND

Microarray sequencing chip is one of the necessary conditions for achieving high-throughput sequencing. In the currently used DNA Nano Ball (DNB) sequencing technology, it requires fixing the DNBs on the sequencing chip for the next step of sequencing biochemical reactions. Taking the currently used sequencing chip as an example, each chip has nearly 200 million DNB binding sites on the surface thereof. In order to stably fix DNBs on the binding sites, the surface of the sequencing chip needs to be aminated. In addition, the regions other than the non-binding sites on the chip surface need to be further treated to minimize non-specific adsorption, reduce signal of background, and improve sequencing quality. Therefore, efficient and low-cost preparation of sequencing chips with microarray is one of the basic tasks to achieve high-quality sequencing.

The current preparation steps of sequencing chips mainly include: firstly, a patterned photoresist layer containing nano-arrays is prepared on a silicon wafer by a semiconductor process, the patterned layer may contain multiple identical unit structures, and each unit can form one sequencing chip; then the wafer with the patterned layer is subjected to chemical vapor deposition processing to form an amination layer on the functional region of the wafer; then by the assembly process, the wafer is divided into single chips, which will be assembled into sequencing chip to be tested.

However, the adsorption of DNBs on the surface of the sequencing chip is not sufficient for the multiple cycle sequencing, so a new DNB loading and fixation method needs to be developed.

### SUMMARY

This disclosure is based on the inventor's discovery and understanding of the following issues:

The current production method of sequencing chips relies on the difference in the interaction with phi29 DNA polymerase and DNBs between transition metal oxide and SiO₂, for specifically adsorbing DNBs on the surface of the transition metal oxide. However, these interactions cannot stabilize the DNA nano balls on the chip surface. During the sequencing process, some DNA nano balls are washed away by the sequencing reagent. An existing solution uses a layer of protein membrane to protect the DNA nano balls adsorbed on the aminated surface, but this solution is not suitable for transition metal oxide chips because of the limited interaction strength of the protein membrane with the transition metal oxide.

For the DNB array chip of the transition metal oxide, the inventors developed a new method for fixing DNBs by using a phosphonic acid polymer. Based on the chemical reactions between the phosphonic acid polymer and the transition metal oxide, DNB nano balls can be stable on the surface of the transition metal oxide and can resist the flushing of sequencing reagents.

In a first aspect of the present disclosure, the present disclosure proposes a sequencing chip. According to embodiments of the present disclosure, the sequencing chip includes: a chip main body, nucleic acids, and a phosphonic acid polymer film. The chip main body includes a plurality of chip particles arranged in a same layer, and the plurality of chip particles are obtained by cutting a chip matrix along cutting lines of a wafer layer. The chip matrix includes: the wafer layer having the cutting lines uniformly distributed thereon; a first silicon oxide layer made of silicon oxide and formed on an upper surface of the wafer layer; and a transition metal oxide layer made of a transition metal oxide and formed on an upper surface of the first silicon oxide layer. The nucleic acids are fixed on the transition metal oxide layer. The phosphonic acid polymer film is made up of a polyphosphonic acid polymer and is formed on an upper surface of the transition metal oxide layer.

Unless otherwise specified, the term "chip matrix" used in the present disclosure refers to a sequencing chip unit that can be cut apart into chip particles, for example, the chip matrix according to the embodiment of the present disclosure can be cut apart into chip particles that can be combined in the same direction and in the same layer to form the main body of the sequencing chip.

In the sequencing chip according to the embodiments of the present disclosure, the hydrophilic phosphonic acid polymer film is used instead of the existing protein membrane to fix the DNA nano balls. The polymer film can lie firmly on the surface of the transition metal oxide by the chemical reaction between phosphonic acid and the transition metal oxide, so as to fix the nucleic acids to be sequenced. Since the polymer film has a macromolecular characteristic, it will form a mesh structure after being adsorbed to the surface, thereby ensuring the stability of the nucleic acids on the chip surface.

In a second aspect of the present disclosure, the present disclosure proposes a method for preparing the sequencing chip. According to embodiments of the present disclosure, the method includes: 1) performing surface modification on the wafer layer to obtain the chip matrix, wherein the surface modification comprises: processing a surface of the wafer layer with a transition metal oxide to form the transition metal oxide layer, the wafer layer comprising a first silicon oxide layer made of silicon oxide on an upper surface thereof, the transition metal layer being formed on the upper surface of the first silicon oxide layer, and the wafer layer having the cutting lines uniformly distributed thereon; 2) cutting the chip matrix along the cutting lines of the wafer layer to obtain chip particles; 3) obtaining the chip main body by assembling the chip particles; 4) fixing DNA nano balls on the chip main body; 5) incubating the chip main body fixed with the DNA nano balls in a buffer solution of the polyphosphonic acid polymer to obtain the sequencing chip. According to the method of the embodiments of the present disclosure, the operation is simple, the prepared sequencing chip has a high yield, and in the obtained sequencing chip, the DNA nano balls are highly stably fixed on the chip surface, which can meet the requirements of multiple cycle sequencing.

In a third aspect of the present disclosure, the present disclosure proposes a sequencing method. According to embodiments of the present disclosure, the method includes: performing sequencing using the sequencing chip defiend as above or prepared according to the above-described method. According to the method of the embodiments of the present disclosure, multiple cycle sequencing can be performed, with high accuracy of the sequencing results and low cost.

### BRIEF DESCRIPTION OF DRAWINGS

FIGS. 1 to 5 are sectional views of procedures of the preparation process of a sequencing chip including a patterned transition metal oxide layer structured in an array of "spots" described in one aspect of the present disclosure, wherein,
FIG. 1 is a sectional view of a wafer structure 1-10 in which a layer of transition metal oxide film structured in an array of "spots" is formed on a wafer having a silicon oxide layer on a surface thereof, according to an example of the present disclosure;
FIG. 2 is a sectional view of multiple single chips 1-20 formed by cutting the wafer structure 1-10 in FIG. 1, according to an example of the present disclosure;
FIG. 3 is a sectional view of a sequencing chip 1-30 formed by assembling the chip in FIG. 2, according to an example of the present disclosure;
FIG. 4 is a sequencing chip 1-40 after surface functional modification of the sequencing chip 1-30 in FIG. 3, according to an example of the present disclosure;
FIG. 5A is a sectional view of a sequencing chip 1-50A containing a DNB array formed after loading of DNBs on the surface-modified sequencing chip 1-40 in FIG. 4, according to an example of the present disclosure;
FIG. 5B is a sectional view of a sequencing chip 1-50B in which a DNB array can be formed more concisely without surface modification, according to an example of the present disclosure;
FIG. 5C shows a relationship between fluorescence signal intensity and silicon oxide layer thickness according to an example of the present disclosure;
FIG. 5D shows a relationship between fluorescence signal intensity and transition metal oxide thickness according to an example of the present disclosure ('spot' structure);
FIGS. 6 to 11 are sectional views of procedures of a preparation process of a sequencing chip having a patterned transition metal oxide layer with an array of "well" structures described in another aspect of the present disclosure, wherein,

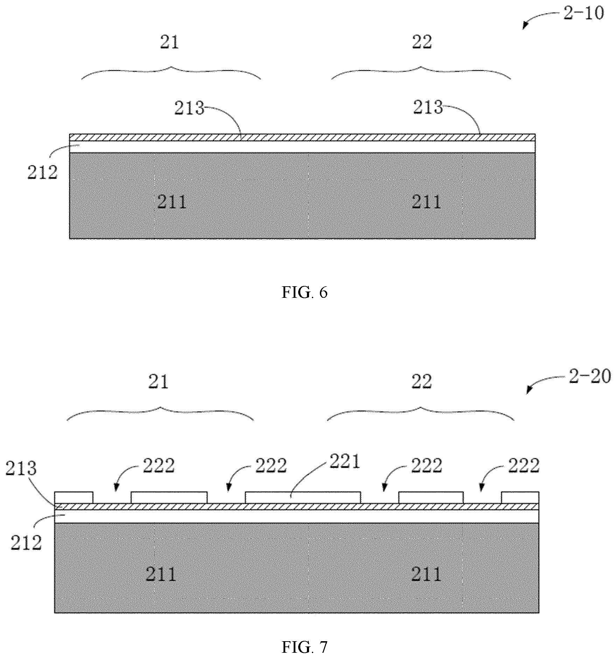

FIG. 6 is a sectional view of a wafer structure 2-10 in which a transition metal oxide layer film is formed on a wafer having a silicon oxide layer on a surface thereof according to an example of the present disclosure;
FIG. 7 is a sectional view of a wafer 2-20 formed after a silicon oxide layer having an array of "well" structures is formed on the wafer structure 2-10 having the transition metal oxide layer film as shown in FIG. 6, according to an example of the present disclosure;

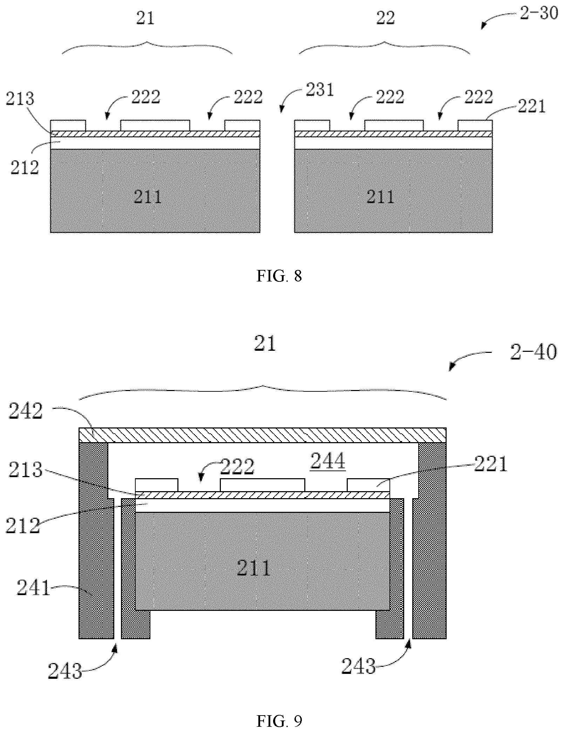

FIG. 8 is a sectional view of multiple single chips 2-30 formed by cutting the wafer structure 2-20 having an array of well structures as shown in FIG. 7, according to an example of the present disclosure;
FIG. 9 is a sectional view of a sequencing chip 2-40 formed by assembling the single chip in FIG. 8 according to an example of the present disclosure;

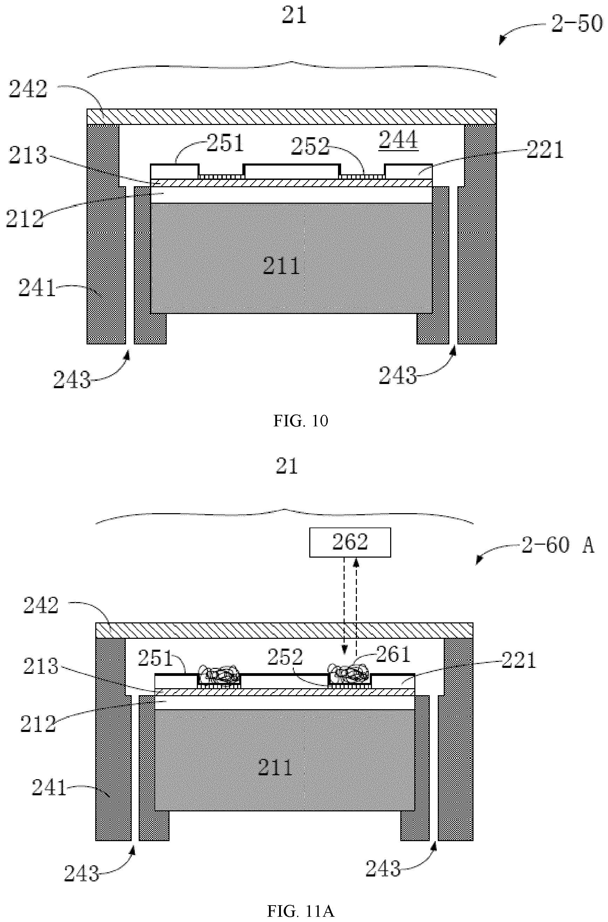

FIG. 10 is a sectional view of a sequencing chip 2-50 formed after surface functional modification of the sequencing chip shown in FIG. 9 according to an example of the present disclosure;
FIG. 11A is a sectional view of a sequencing chip 2-60A with a DNB array formed after loading of DNBs on the surface functionally modified sequencing chip 2-50 shown in in FIG. 10 according to an example of the present disclosure;

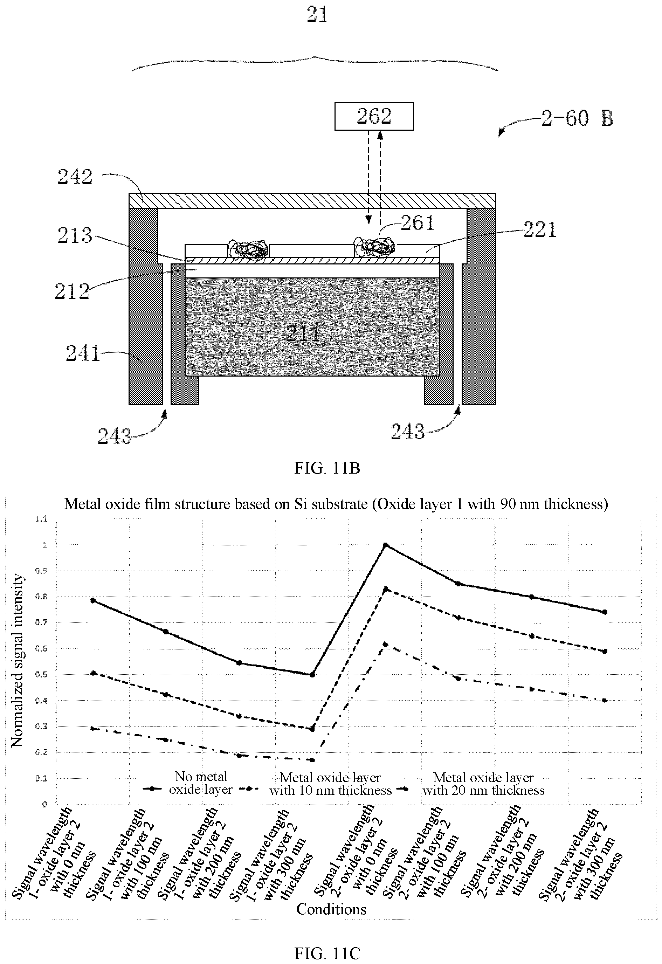

FIG. 11B is a sectional view of a sequencing chip 2-60B in which a DNB array can be formed more concisely without surface modification according to an example of the present disclosure;
FIG. 11C shows a relationship between a fluorescence signal and a second oxide layer of different well structures based on a silicon substrate according to an example of the present disclosure;

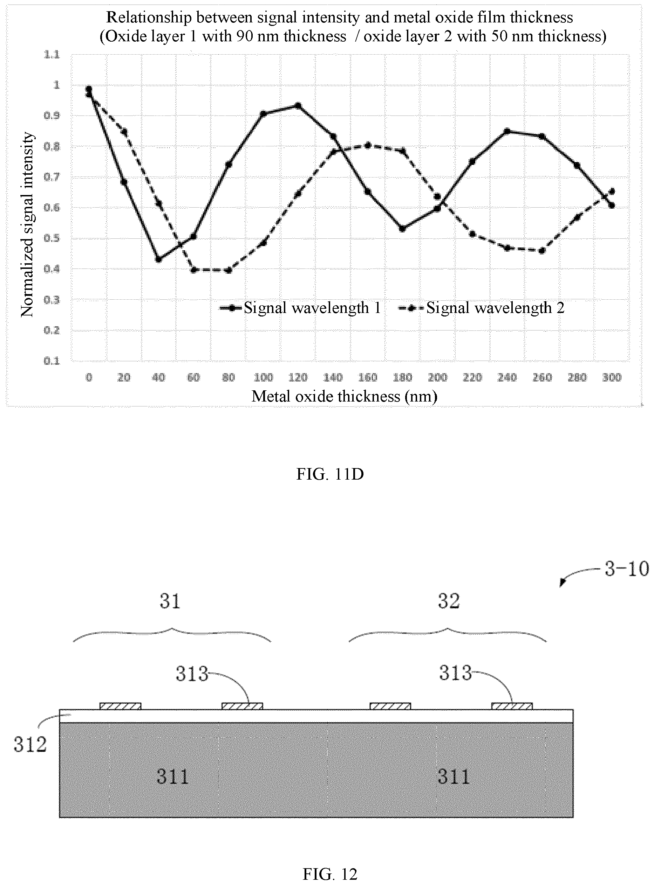

FIG. 11D shows a relationship between fluorescence signal intensity and transition metal oxide thickness according to an example of the present disclosure;
FIGS. 12 to 17 are sectional views of procedures of a preparation process of a sequencing chip having a patterned transition metal oxide layer having another array of well structures described in another aspect of the present disclosure, wherein,
FIG. 12 is a sectional view of a wafer structure 3-10 in which a transition metal oxide layer structured in an array of "spots" is formed on a wafer having a silicon oxide layer on a surface thereof according to an example of the present disclosure;

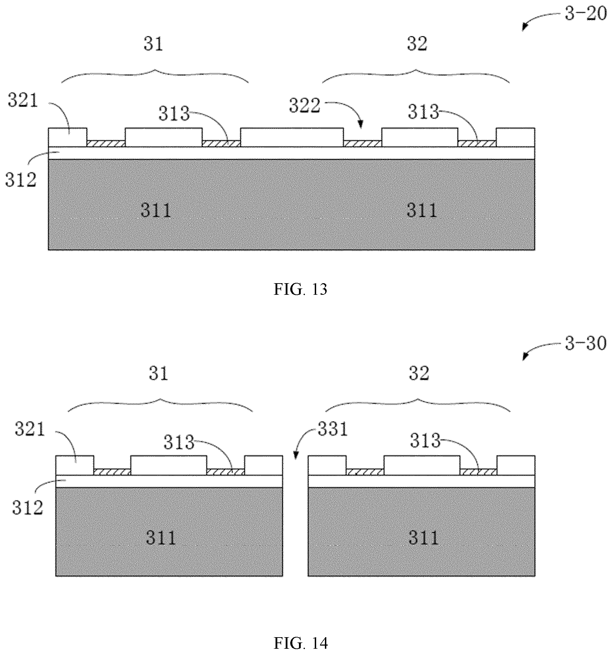

FIG. 13 is a sectional view of a wafer 3-20 formed after a silicon oxide layer having an array of well structures is formed on the wafer structure 3-10 having the transition metal oxide layer film shown in FIG. 12, according to an example of the present disclosure;
FIG. 14 is a sectional view of multiple single chips 3-30 formed by cutting the wafer structure 3-20 having an array of well structures shown in FIG. 13 according to an example of the present disclosure;

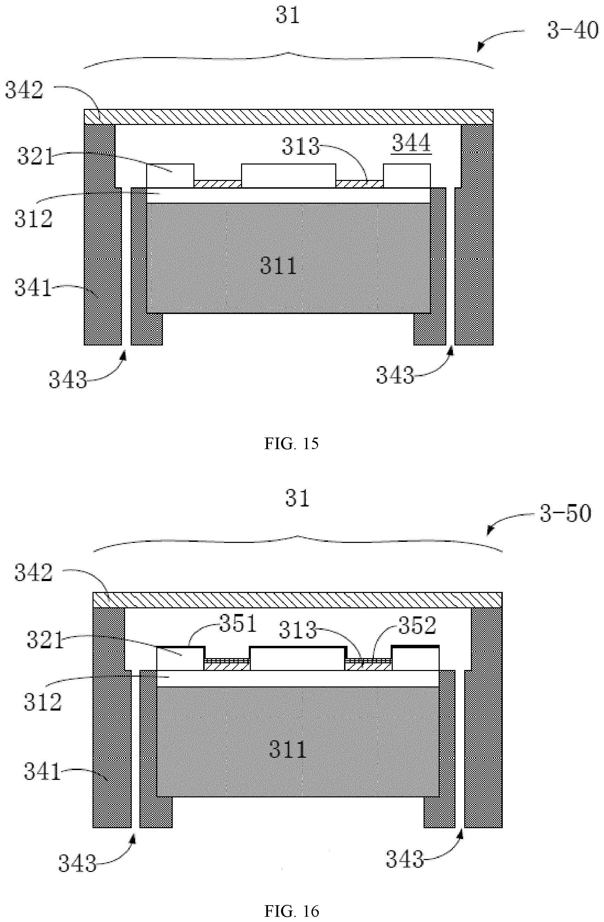

FIG. 15 is a sectional view of a sequencing chip 3-40 formed by assembling the single chip in FIG. 14 according to an example of the present disclosure;
FIG. 16 is a sectional view of a sequencing chip 3-50 formed after surface functional modification of the sequencing chip shown in FIG. 15 according to an example of the present disclosure;

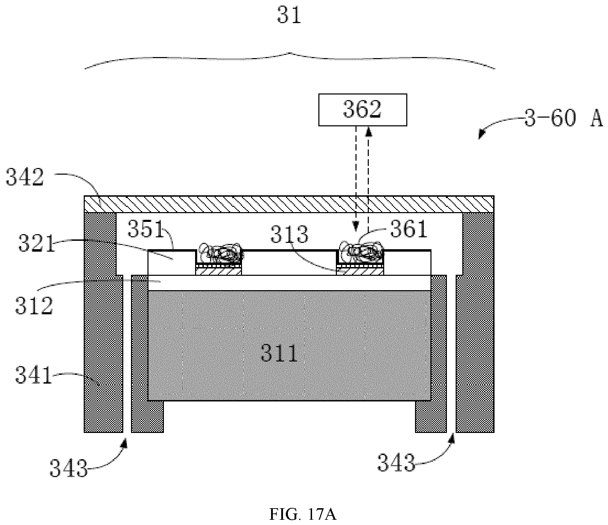

FIG. 17A is a sectional view of a sequencing chip 3-60A having a DNB array formed after loading of DNBs on the surface functionally modified sequencing chip 3-50 shown in FIG. 16, according to an example of the present disclosure;

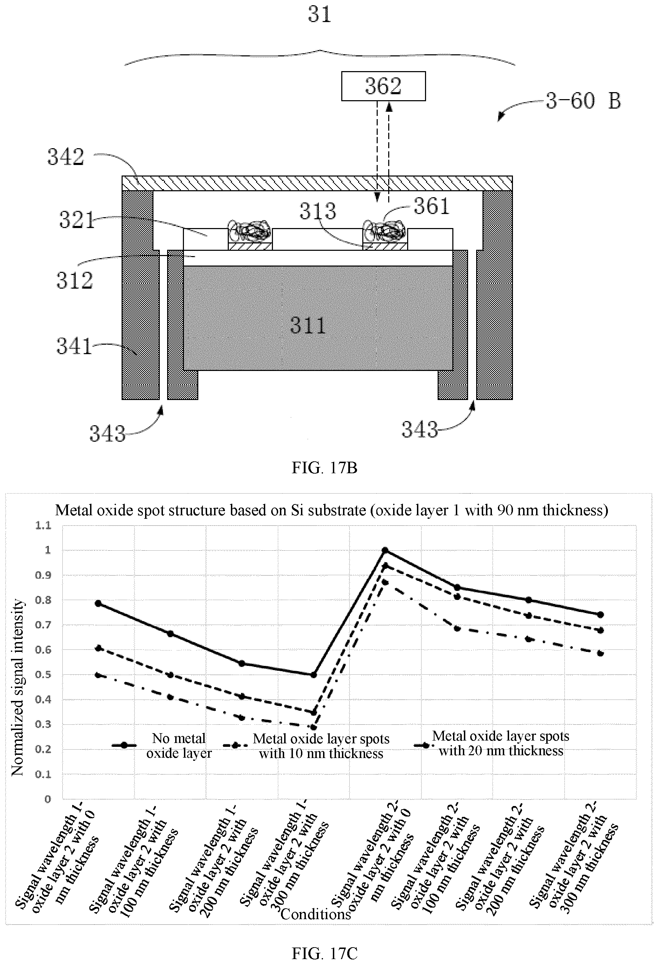

FIG. 17B is a sectional view of a sequencing chip 3-60B in which a DNB array can be formed more concisely without surface modification according to an example of the present disclosure;
FIG. 17C shows another relationship between a fluorescence signal and a second oxide layer of different well structures based on a silicon substrate according to an example of the present disclosure;

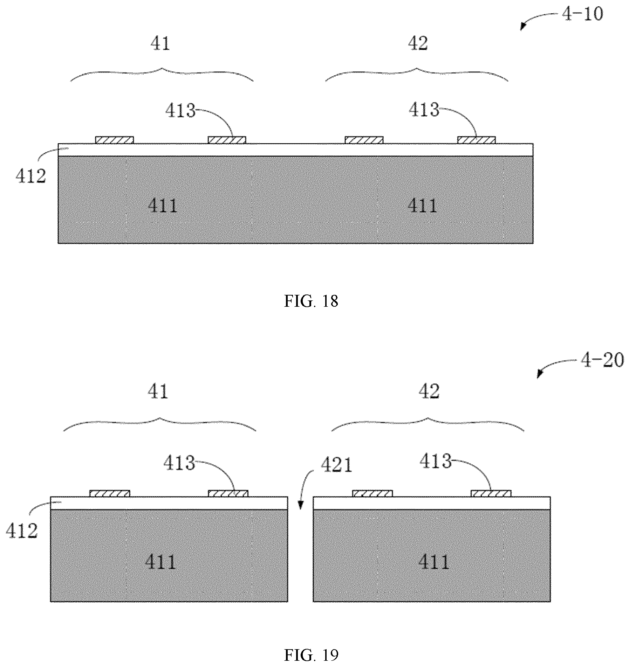

FIGS. 18 to 22 are sectional views of procedures of a preparation process of a back-illuminated sequencing chip having a patterned transition metal oxide layer structured in an array of "spots" described in another aspect of the present disclosure;
FIG. 18 is a sectional view of a wafer structure 4-10 in which a transition metal oxide layer film structured in an array of "spots" is formed on a wafer having a silicon oxide layer on a surface thereof according to an example of the present disclosure;
FIG. 19 is a sectional view of multiple single chips 4-20 formed by cutting the wafer structure 4-10 having an array of well structures shown in FIG. 18 according to an example of the present disclosure;

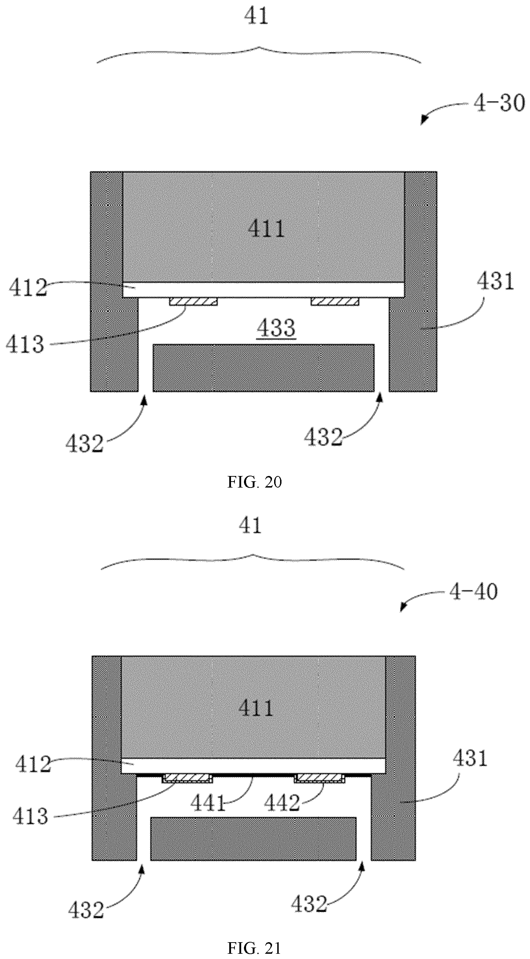

FIG. 20 is a sectional view of a sequencing chip 4-30 formed by assembling the single chip 4-20 in FIG. 19 according to an example of the present disclosure; in this assembling process, the the chip is assembled with a frame with the patterned layer facing downward, so that an excitation light source illuminates DNBs from the back of the chip through a quartz or glass substrate and the signals are collected by a camera;
FIG. 21 is a sectional view of a sequencing chip 4-40 formed after surface functional modification of the sequencing chip shown in FIG. 20 according to an example of the present disclosure;

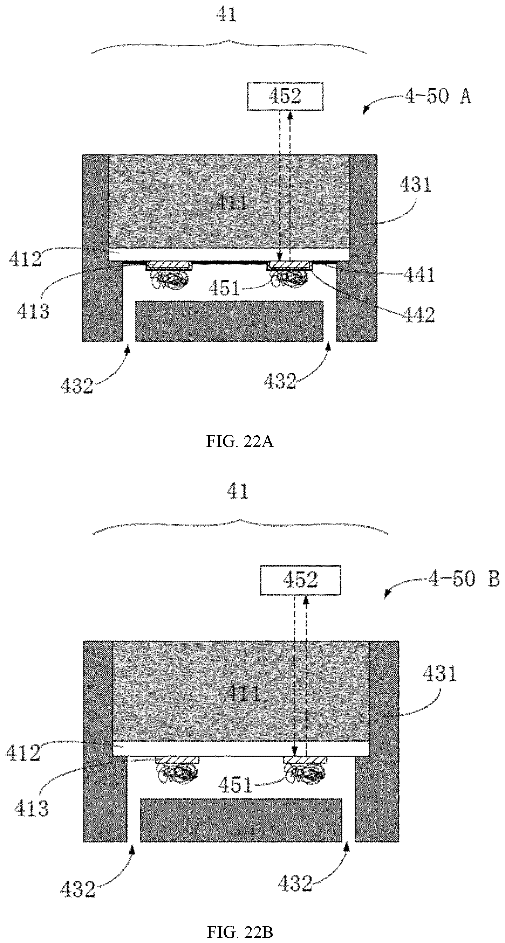

FIG. 22A is a sectional view of a sequencing chip 4-50A having a DNB array formed after loading of DNBs on the surface functionally modified sequencing chip 4-40 shown in FIG. 21 according to an example of the present disclosure;
FIG. 22B is a sectional view of a sequencing chip 4-50B in which a DNB array can be formed more concisely without surface modification according to an example of the present disclosure;

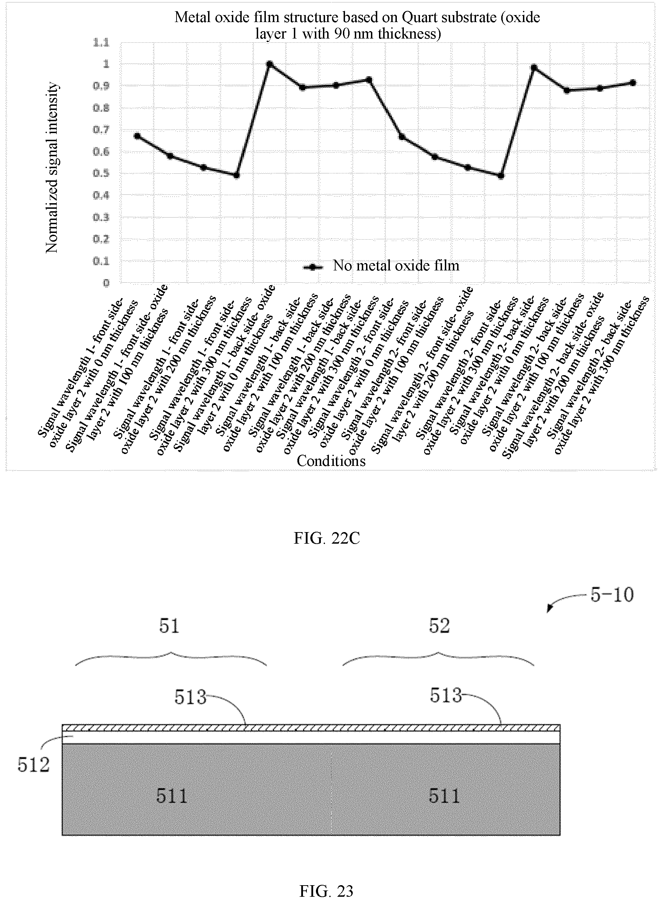

FIG. 22C shows a relationship between a fluorescence signal and a second oxide layer based on a quartz substrate according to an example of the present disclosure (back-illuminated, "spot" structure);
FIGS. 23 to 28 are sectional views of procedures of a preparation process of a back-illuminated sequencing chip having a patterned transition metal oxide layer having an array of "well" structures described in another aspect of the present disclosure;
FIG. 23 is a sectional view of a wafer structure 5-10 in which a transition metal oxide layer film is formed on a wafer having a silicon oxide layer on a surface thereof according to an example of the present disclosure;

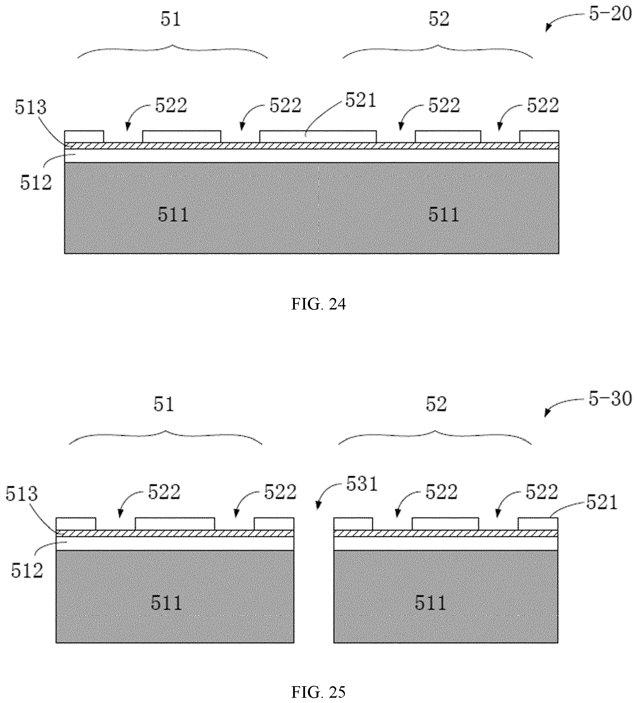

FIG. 24 is a sectional view of a wafer structure 5-20 formed after a silicon oxide layer having an array of "well" structures is formed on the wafer structure 5-10 having a transition metal oxide layer film shown in FIG. 23 according to an example of the present disclosure;
FIG. 25 is a sectional view of multiple single chips 5-30 formed by cutting the wafer structure 5-20 having an array of "well" structure shown in FIG. 24 according to an example of the present disclosure;

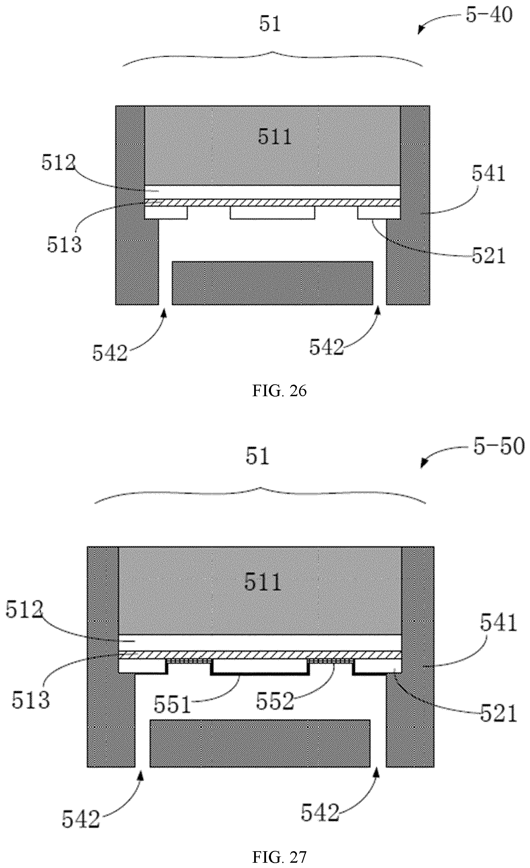

FIG. 26 is a sectional view of a sequencing chip 5-40 formed by assembling the single chip in FIG. 25 according to an example of the present disclosure; in this assembly process, the chip is assembled with the frame with the patterned layer of the chip facing downward, so that the excitation light source illuminates DNBs from the back of the chip through a quartz or glass substrate and signals are collected by a camera;
FIG. 27 is a sectional view of a sequencing chip 5-50 formed after surface functional modification of the sequencing chip shown in FIG. 26 according to an example of the present disclosure;

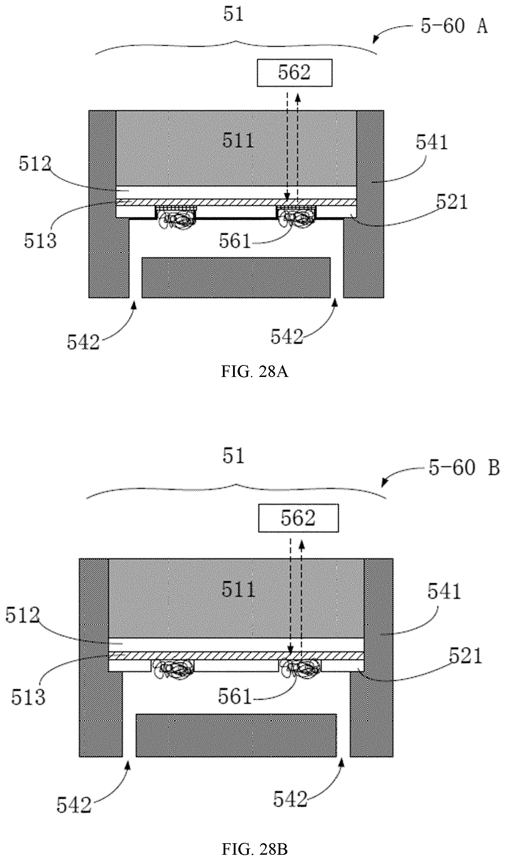

FIG. 28A is a sectional view of a sequencing chip 5-60A with a DNB array formed after loading of DNBs on the surface functionally modified sequencing chip 5-50 in FIG. 27 according to an example of the present disclosure;
FIG. 28B is a sectional view of a sequencing chip 5-60B in which a DNB array can be formed more concisely without surface modification according to an example of the present disclosure;

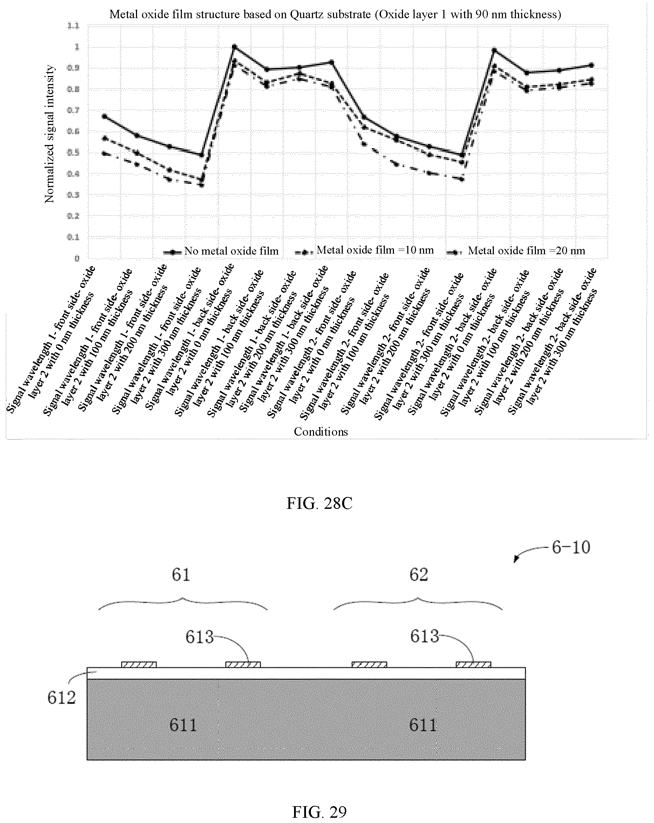

FIG. 28C shows a relationship between a fluorescence signal and a second oxide layer of different "well" structures based on a quartz substrate according to an example of the present disclosure (back-illuminated, "well" structure);
FIGS. 29 to 34 are sectional views of procedures of a preparation process of a back-illuminated sequencing chip having a patterned transition metal oxide layer having an array of "well" structures described in another aspect of the present disclosure,
FIG. 29 is a sectional view of a wafer structure 6-10 in which a transition metal oxide layer 613 structured in an array of "spots" is formed on a wafer 611 having a silicon oxide layer 612 on a surface thereof according to an example of the present disclosure;

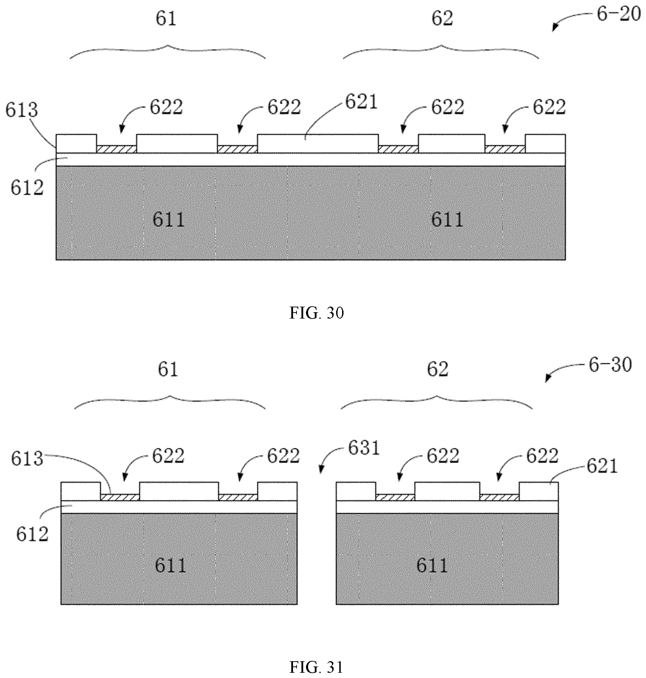

FIG. 30 is a sectional view of a wafer structure 6-20 formed after a silicon oxide layer having an array of "well" structures is formed on the wafer structure 6-10 having a transition metal oxide layer film shown in FIG. 29 according to an example of the present disclosure;
FIG. 31 is a sectional view of multiple single chips 6-30 formed by cutting the wafer structure 6-20 having an array of "well" structure shown in FIG. 30 according to an example of the present disclosure;

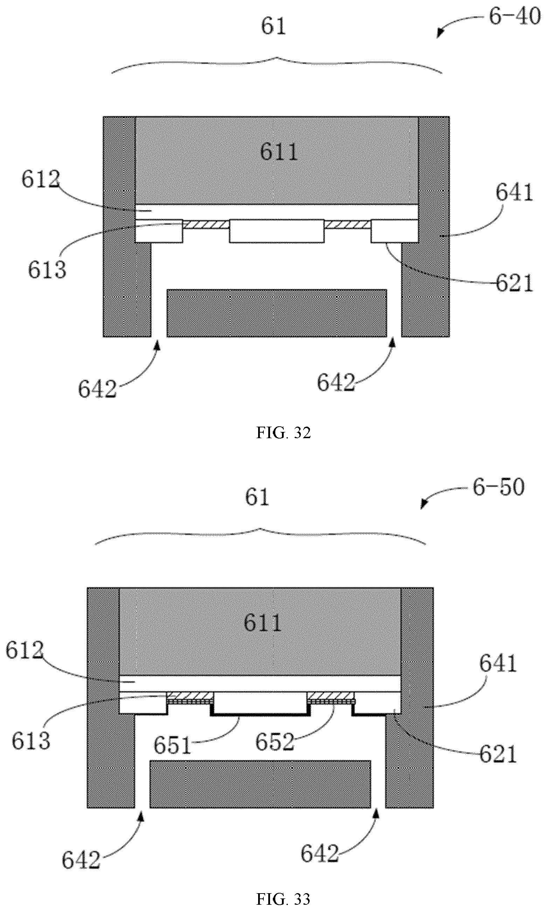

FIG. 32 is a sectional view of a sequencing chip 6-40 formed by assembling the single chip 6-30 in FIG. 31 according to an example of the present disclosure; in this assembly process, the chip is assembled with the frame with the patterned layer of the chip facing downward, so that the excitation light source illuminates DNBs from the back of the chip through a quartz or glass substrate and the signals are collected by a camera;
FIG. 33 is a sectional view of a sequencing chip 6-50 formed after surface functional modification of the sequencing chip shown in FIG. 32 according to an example of the present disclosure;

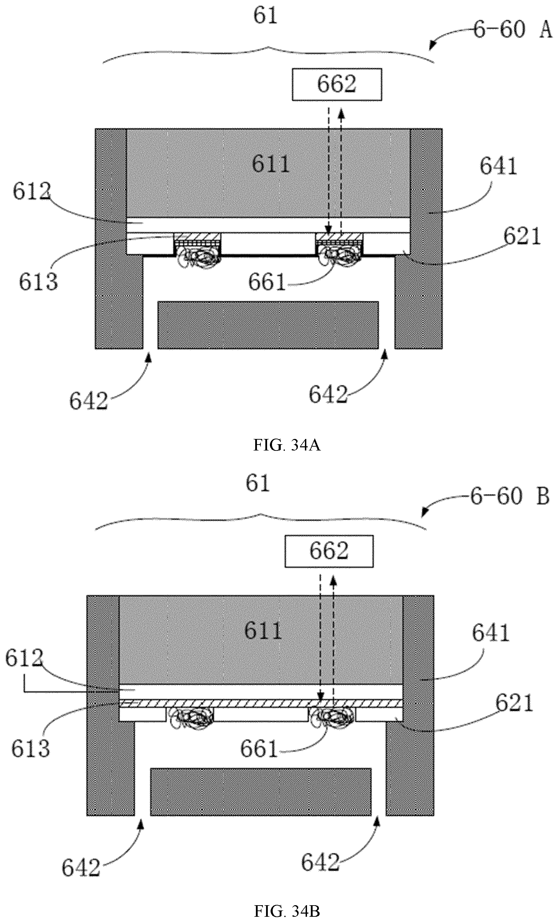

FIG. 34A is a sectional view of a sequencing chip 6-60A with a DNB array formed after loading of DNBs on the surface functionally modified sequencing chip 6-50 in FIG. 33 according to an example of the present disclosure;
FIG. 34B is a sectional view of a sequencing chip 6-60B in which a DNB array can be formed more concisely without surface modification according to an example of the present disclosure;

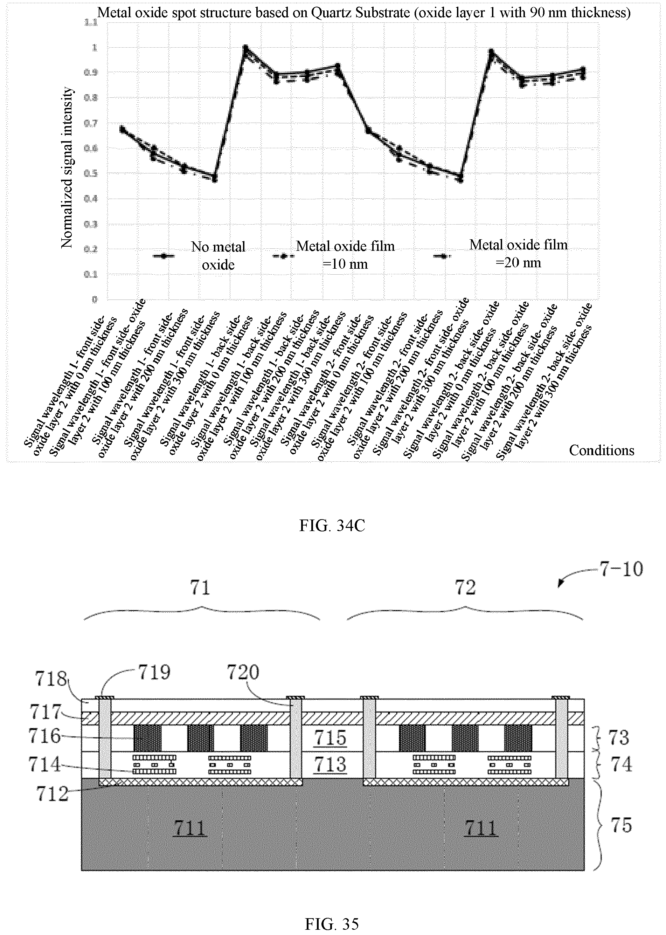

FIG. 34C shows another relationship between a fluorescence signal and a second oxide layer of different "well" structures based on a quartz substrate according to an example of the present disclosure (another back-illuminated "well" structure);
FIGS. 35 to 41 are sectional views of procedures of a preparation process of a sequencing chip having a patterned transition metal oxide layer structured in an array of "spots" or having an array of "well" structures on a CMOS image sensor wafer described in another aspect of the present disclosure,
FIG. 35 is a sectional view of a CMOS image sensor wafer 7-10 with a surface oxide layer according to an example of the present disclosure;

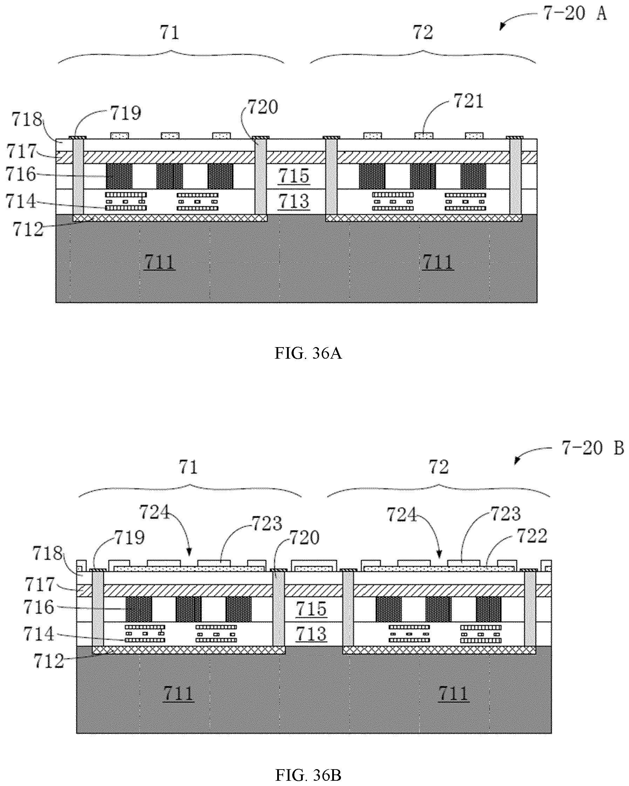

FIG. 36A is a sectional view of a wafer structure 7-20A formed after a patterned transition metal oxide layer structured in an array of "spots" is formed on the CMOS wafer 7-10 shown in FIG. 35 according to an example of the present disclosure;
FIG. 36B is a sectional view of a wafer structure 7-20B formed after a patterned transition metal oxide layer film having an array of "well" structures is formed on the CMOS wafer 7-10 shown in FIG. 35 according to an example of the present disclosure;

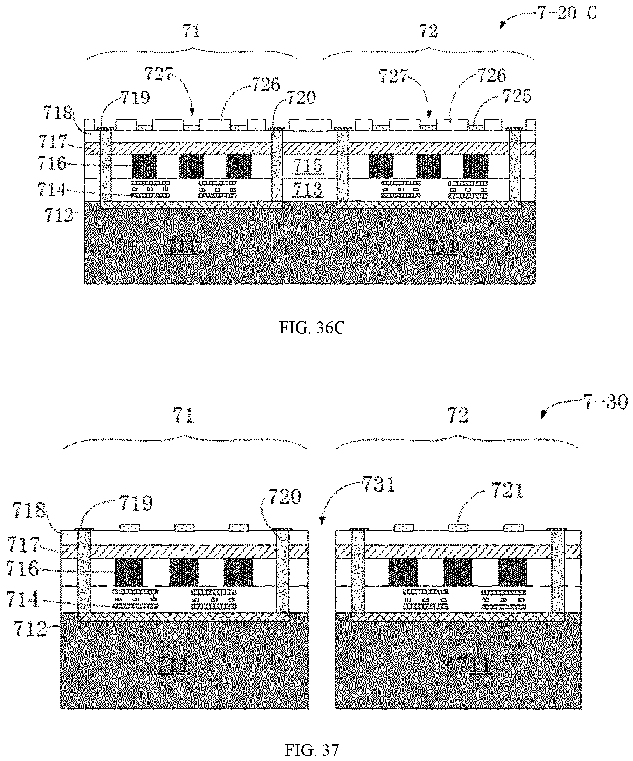

FIG. 36C is a sectional view of a wafer structure 7-20C formed after another patterned transition metal oxide layer having an array of "well" structures is formed on the CMOS wafer 7-10 shown in FIG. 35 according to an example of the present disclosure;
FIG. 37 is a sectional view of multiple single chips 7-30 formed by cutting the CMOS wafer 7-20 having the patterned transition metal oxide layer shown in FIG. 36 according to an example of the present disclosure;

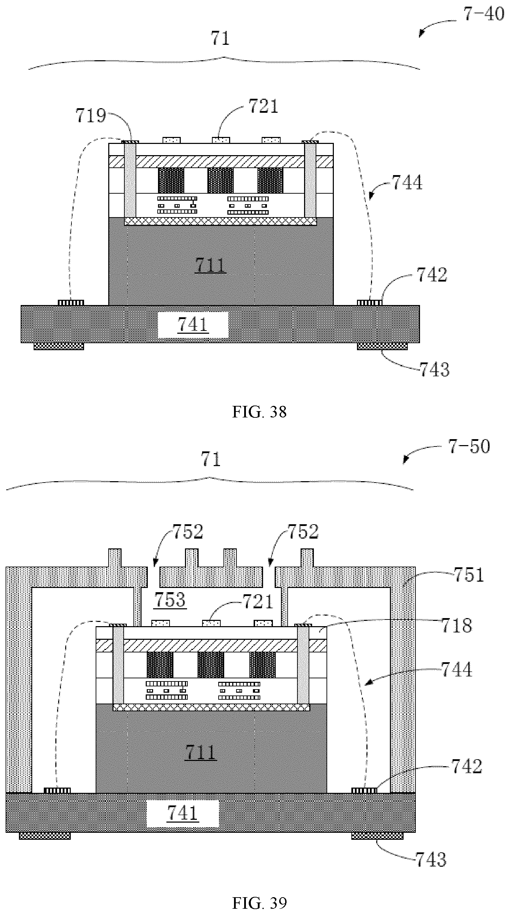

FIG. 38 is a sectional view of a chip structure 7-40 formed after chip attachment and wire bonding of the single chips shown in FIG. 37 according to an example of the present disclosure;
FIG. 39 is a sectional view of a chip structure 7-50 formed after attachment of a lid structure for the chip 7-50 which has been subjected to chip attachment and wire bonding shown in FIG. 38 according to an example of the present disclosure;

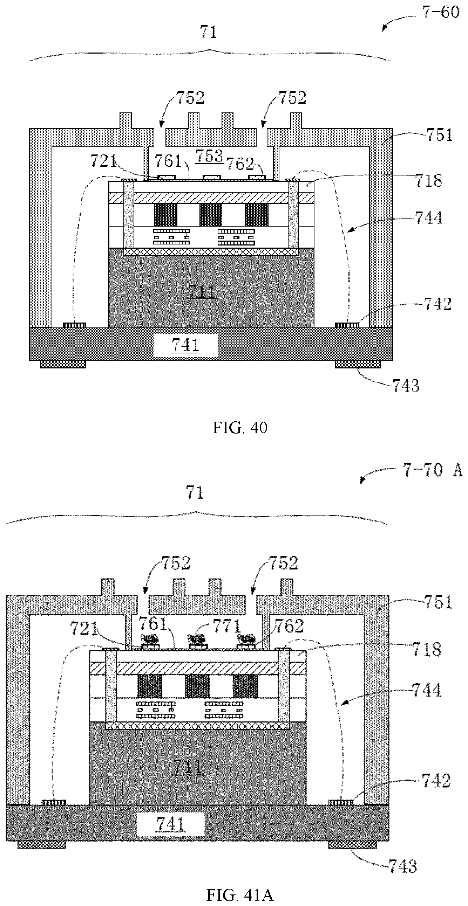

FIG. 40 is a sectional view of a sequencing chip 7-60 formed after surface functional modification of the sequencing chip shown in FIG. 39 according to an example of the present disclosure;
FIG. 41A is a sectional view of a sequencing chip 7-70A with a DNB array formed after loading of DNBs on the surface functionally modified sequencing chip 7-60 in FIG. 40 according to an example of the present disclosure;

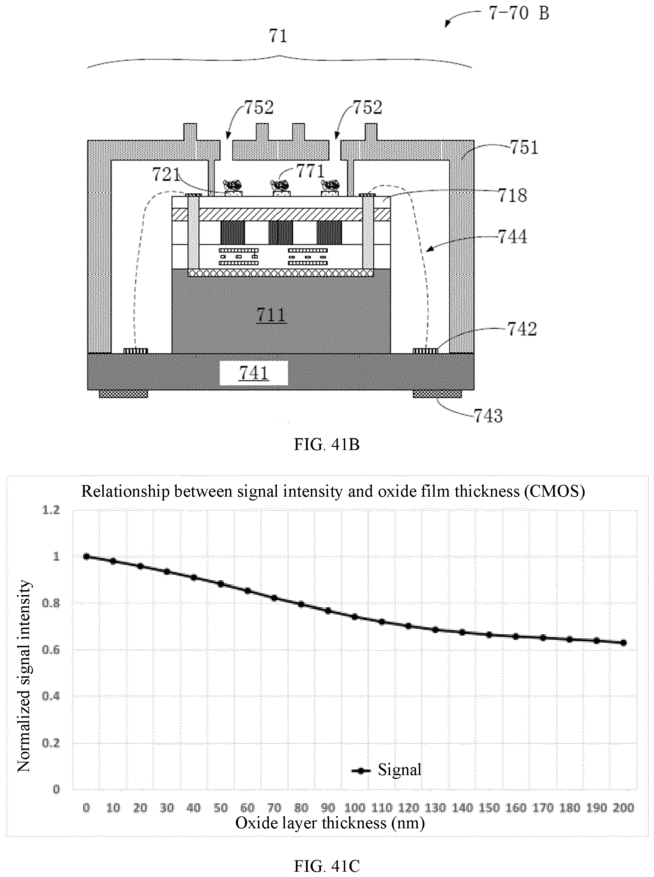

FIG. 41B is a sectional view of a sequencing chip 7-70B in which a DNB array can be formed more concisely without surface modification according to an example of the present disclosure;
FIG. 41C shows a relationship between the fluorescence intensity and the thickness of the top oxide layer based on the CMOS structure according to an example of the present disclosure;

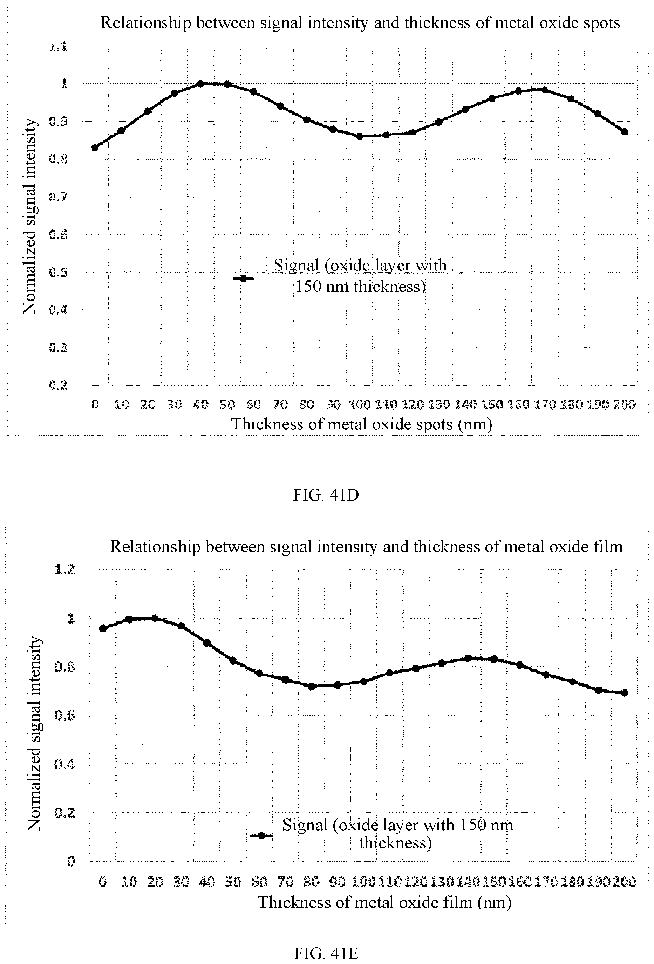

FIG. 41D shows a relationship between the fluorescence intensity and the thickness of the "spot" metal oxide layer based on the CMOS structure according to an example of the present disclosure;
FIG. 41E shows a relationship between the fluorescence intensity and the thickness of the film metal oxide layer based on the CMOS structure according to an example of the present disclosure;

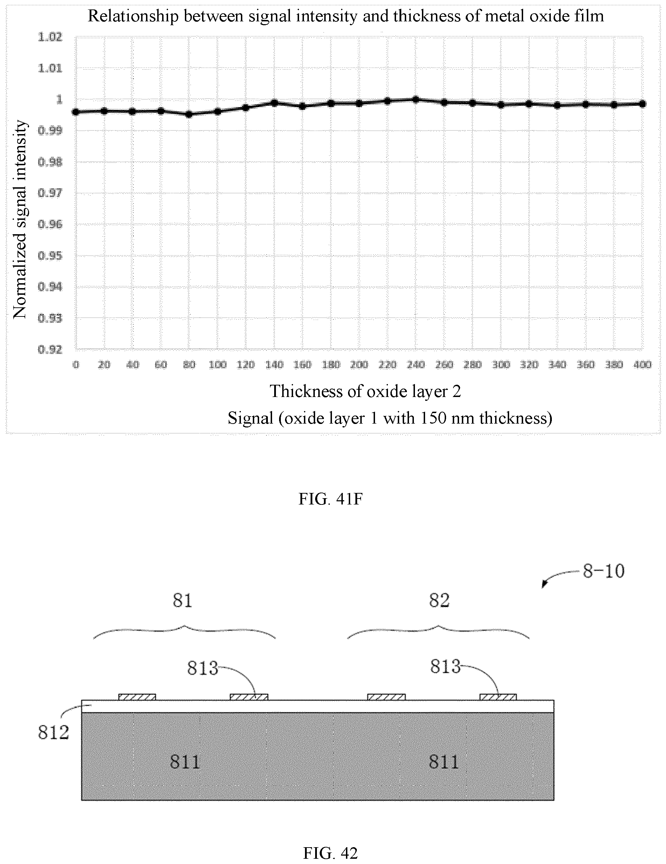

FIG. 41F shows a relationship between the fluorescence intensity and the thickness of the second oxide layer based on the CMOS structure according to an example of the present disclosure (the film metal oxide layer);
FIG. 42 is a sectional view of a wafer structure 8-10 having a transition metal oxide layer structured in an array of "spots" according to an example of the present disclosure;

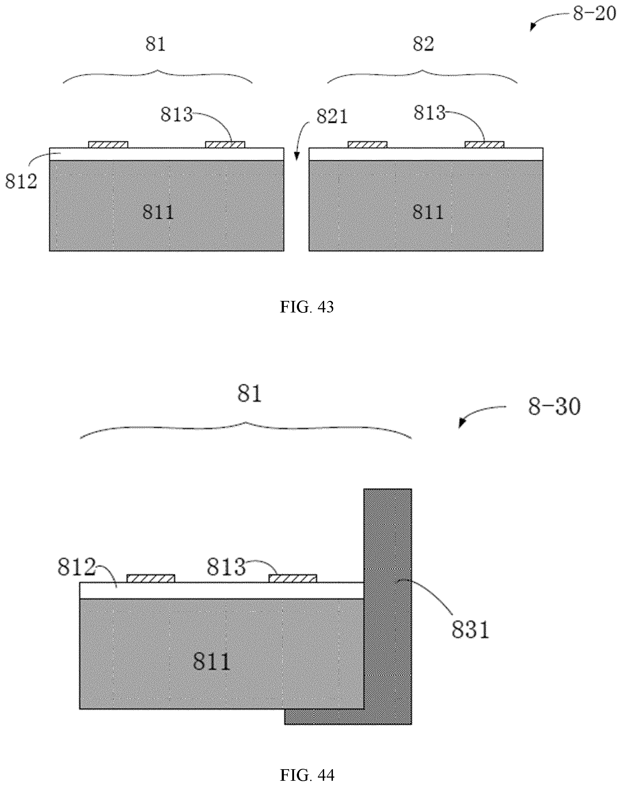

FIG. 43 is a sectional view of a wafer structure 8-20 with multiple single chips 81 and 82 formed after cutting the wafer structure 8-10 shown in FIG. 42 according to an example of the present disclosure;
FIG. 44 is a sectional view of a reusable sequencing chip 8-30 formed by assembling the single chip 81 or 82 formed in FIG. 43 with a handle structure 831 according to an example of the present disclosure;

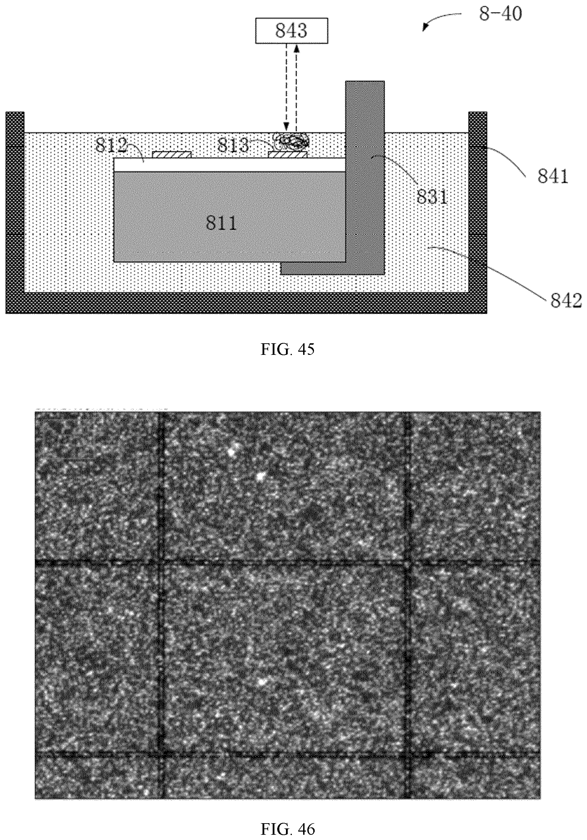

FIG. 45 is a schematic diagram showing that the assembled sequencing chip shown in FIG. 44 is immersed in a container 841 filled with a reagent 842 according to an example of the present disclosure;
FIG. 46 is a fluorescence image of a transition metal oxide chip in which the surface is not modified, but the DNB loading conditions are changed according to an example of the present disclosure;

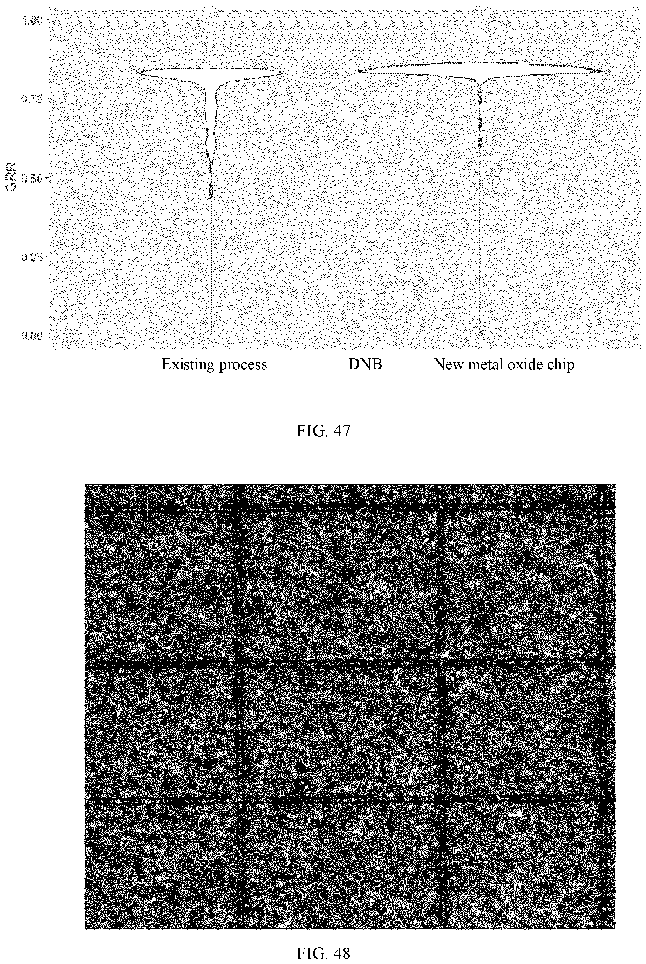

FIG. 47 is a diagram showing the sequencing results on the Zebra platform according to an example of the present disclosure;
FIG. 48 is a fluorescence image after loading of DNBs subsueqnt to a selective amination modification on the surface of the transition metal oxide chip according to an example of the present disclosure;

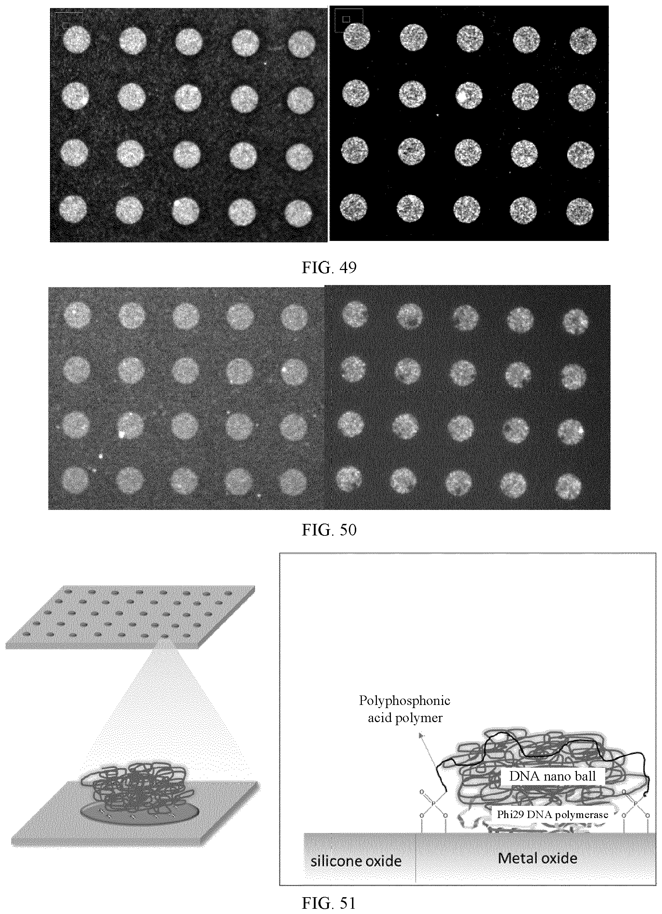

FIG. 49 is a fluorescent image after loading of DNBs subsequent to amination of the transition metal oxide region and copolymer modification of the silicon dioxide non-functional region according to an example of the present disclosure. Left: control group; right: experimental group;
FIG. 50 is a fluorescence image of effect detection of further modification of the non-functional region by using a silane coupling agent containing polyethylene glycol according to an example of the present disclosure. Left: control group; right: experimental group;
FIG. 51 is a diagram showing priciples for fixing the DNA nano ball by using the polymer according to an example of the present disclosure;

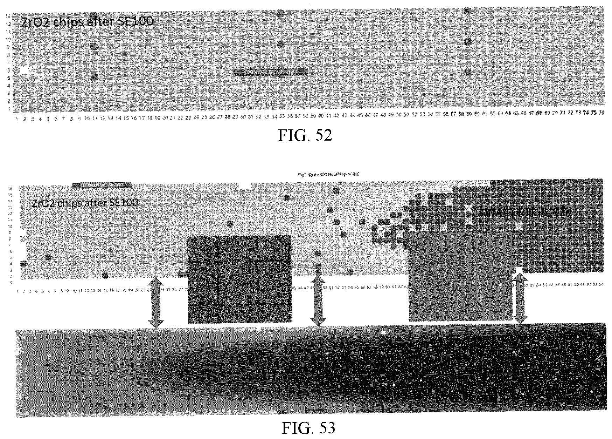

FIG. 52 is a result diagram of stability performance of polymer-fixed DNA nano balls during sequencing according to an example of the present disclosure; and
FIG. 53 is a result diagram of stability performance of protein membrane-fixed DNA nano balls during sequencing according to an example of the present disclosure.

### Reference:

1-10: wafer structure,
11 and 12: single chips on wafer,
111: substrate structure of wafer,
112: silicon oxide layer,
113: patterned transition metal oxide layer (i.e., transition metal oxide "spots"),
1-20: multiple single wafer structures,
121: cutting slot,
1-30: sequencing chip assembled by single chips,
131: frame structure,
132: cover glass,
133: liquid inflow-outflow opening,
134: fluid channel,
1-40: sequencing chip formed after functional surface modification,
141: amino group,
142: polyethylene glycol molecular layer,
1-50A: sequencing chip containing a DNB array formed after loading of DNBs,
1-50B: sequencing chip in which a DNB array is formed,
151: DNB sample,
152: light source and camera,
2-10: wafer structure formed after a silicon oxide layer and a transition metal oxide layer are formed on raw wafer,
21 and 22: single chips,
211: wafer substrate,
212: silicon oxide layer,
213: transition metal oxide layer,
2-20: wafer formed after a silicon oxide layer patterned with "well" structures is formed on the wafer containing the transition metal oxide layer,
221: silicon oxide layer,
222: discretely distributed array of "well" structures,
2-30: multiple single chips formed by cutting the wafer structure,
231: cutting slot,
2-40: sequencing chip formed by assembling single chips,
241: frame,
242: cover glass,
243: liquid inflow-outflow opening,
244: fluid channel,
2-50: sequencing chip formed after surface functional modification,
251: silicon oxide layer,
252: transition metal oxide layer, 2-60A: sequencing chip with DNB array formed after loading of DNBs on the sequencing chip,
2-60B: sequencing chip having a DNB array formed therein;
261: DNB,
262: excitation light source and camera structure,
3-10: wafer structure containing the patterned transition metal oxide layer,
31 and 32: single chip,
311: wafer,
312: silicon oxide layer,
313: transition metal oxide layer,
3-20: wafer structure formed after a silicon oxide layer patterned with "well" structures is formed on the wafer containing the patterned transition metal oxide layer,
321: silicon oxide layer,
322: "well" structure on the silicon oxide layer,
3-30: multiple single chips separated by the cutting slot formed by cutting the wafer structure,
331: cutting slot,
3-40: sequencing chip formed by assembling single chips,
341: frame,
342: cover glass,
343: liquid inflow-outflow opening,
344: fluid channel,
3-50: sequencing chip formed after surface functional modification,
351: silicon oxide layer,
352: transition metal oxide layer,
3-60A: sequencing chip containing a DNB array formed after loading of DNBs on the surface functionally modified sequencing chip,
361: DNB,
362: excitation light source and camera structure,
3-60B: sequencing chip having a DNB array formed therein,
4-10: wafer structure formed after a patterned transition metal oxide layer is formed on a quartz wafer having a silicon oxide layer,
41 and 42: single chip on the wafer,
411: quartz wafer,
412: silicon oxide layer,
413: patterned transition metal oxide layer,
4-20: multiple single chips separated by the cutting slot formed by cutting the wafer,
421: cutting slot,
4-30: sequencing chip formed after packaging of the single chip,
431: frame,
432: liquid inflow-outflow opening,
433: fluid channel,
4-40: sequencing chip formed after surface functional modification,
441: silicon oxide layer,
442: transition metal oxide layer,
4-50A: sequencing chip having a DNB array formed after loading of DNBs on the surface functionally modified sequencing chip,
4-50B: sequencing chip having a DNB array formed therein,
451: DNB,
452: excitation light source and camera,
5-10: wafer structure formed after a silicon oxide layer and a transition metal oxide layer are formed on the raw wafer,
51 and 52: single chip on the wafer,
511: structure of wafer substrate,
512: silicon oxide layer,
513: patterned transition metal oxide layer,
5-20: wafer formed after a silicon oxide layer patterned with "well" structures is formed on the wafer containing the transition metal oxide layer,
521: silicon oxide layer,
522: transition metal oxide layer,
5-30: multiple single chips formed by cutting the wafer structure,
531: cutting slot,
5-40: sequencing chip formed by assembling single chips,
541: frame,
542: liquid inflow-outflow opening,
5-50: sequencing chip formed after surface functional modification,
551: silicon oxide layer,
552: transition metal oxide layer,
5-60A: sequencing chip having a DNB array formed after loading of DNBs on the surface functionally modified sequencing chip,
5-60B: sequencing chip having a DNB array formed therein,
561: DNB,
562: excitation light source and camera;
6-10: wafer structure containing the patterned transition metal oxide layer,
61 and 62: single chip on the wafer,
611: wafer,
612: silicon oxide layer,
613: transition metal oxide layer,
6-20: wafer structure formed after a silicon oxide layer patterned with "well" structures is formed on the wafer containing the patterned transition metal oxide layer,
621: silicon oxide layer,
622: "well" structure on the silicon oxide layer,
6-30: multiple single chips separated by the cutting slot formed by cutting the wafer structure,
631: cutting slot,
6-40: sequencing chip formed by packaging a single chip,
641: frame,
642: liquid inflow-outflow opening,
6-50: the sequencing chip formed after surface functional modificaton,
651: silicon oxide layer,
652: transition metal oxide layer,
6-60A: sequencing chip containing a DNB array formed after loading of DNBs on the surface functionally modified sequencing chip,
6-60B: sequencing chip having the DNB array formed therein,
661: DNB,
662: excitation light source and camera,
7-10: CMOS image sensor wafer,
71 and 72: two chips,
73: photosensitive layer,
74: interconnected layer,
75: substrate layer,
711: silicon substrate,
712: CMOS processing circuit layer,
713: dielectric layer,
714: metal wiring,
715: semiconductor material,
716: photosensitive part,
717: dielectric film layer,
718: silicon oxide layer,
719: bonding pad on the chip,
720: interconnected silicon through-hole,
7-20A: CMOS wafer structure formed after a patterned transition metal oxide layer structured in "spots" is formed on the CMOS image sensor wafer,
721: transition metal oxide region,
7-20B: CMOS wafer structure formed after forming a patterned transition metal oxide layer with "well" structure on the CMOS image sensor wafer,
722: transition metal oxide region,
723: silicon oxide region,
724: transition metal oxide region of "well" structure,
7-20C: CMOS wafer structure formed after another patterned transition metal oxide layer with a "well" structure on the CMOS image sensor wafer,
725: transition metal oxide region,
726: silicon oxide region,
727: transition metal oxide region,
7-30: multiple single chips separated by the cutting slot formed by cutting the patterned wafer structure,
731: cutting slot,
7-40: chip structure formed after the chip is subjected to chip attachment and wire bonding,
741: package substrate,
742: bonding pad on the substrate,
743: contact,
744: metal wire,
7-50: sequencing chip formed after a lid structure is attached to the chip structure,
751: lid structure of a support structure,
752: liquid inflow-outflow opening,
753: fluid channel,
7-60: sequencing chip formed after surface functional modification,
761: transition metal oxide region,
762: silicon oxide region,
7-70A: sequencing chip having a DNB array formed by loading of DNBs on the surface functionally modified sequencing chip,
7-70B: sequencing chip having a DNB array formed therein,
771: DNB,
8-10: wafer structure having a transition metal oxide layer structured in an array of "spots",
81 and 82: single chips on the wafer,
811: wafer substrate,
812: silicon oxide layer,
813: transition metal oxide layer structured in "spots",
81 and 82: multiple single chips,
8-20: wafer structure of multiple single chips formed by cutting the wafer structure,
821: cutting line,
8-30: reusable sequencing chip formed by assembling a single chip with a handle structure.
831: handle structure,
8-40: assembled sequencing chip immersed in a container filled with a reagent,
841: container,
842: reagent,
843: excitation light source and camera.

### DESCRIPTION OF EMBODIMENTS

The following examples of the present disclosure are described in details, which are shown in the attached figures. The examples described with reference to the attached figures are exemplary, which are intended to explain the present disclosure, and cannot be understood as restrictions on the present disclosure.

If there are no special instructions, reagents, detection instruments, etc. in the examples can be prepared by oneself or available in the market.

It should be noted that the "transition metal oxide region" described in the present disclosure refers to the region covered by transition metal oxide of the chip matrix surface, and the "silicon oxide region" described in the present disclosure refers to the region covered by silicon oxide of the chip matrix surface.

The term "patterned layer" refers to the shape that the transition metal oxide region and the silicon oxide region alternately exist on the surface of a wafer, including "well" and "spot" structures.

The term "spot" structure refers to that the transition metal oxide region is higher than the silicon oxide region, that is, the transition metal oxide is discretely distributed on the silicon oxide.

The term "the transition metal oxide layer is a continuous layer structure, and the second silicon oxide layer is formed by silicon oxide as a plurality of "well structures" that connected to each other above the upper surface of the transition metal oxide layer" means that the second silicon oxide layer of well structures is located above the upper surface of the transition metal oxide layer, that is, the body of well is silicon oxide, and the transition metal oxide is at the recessed portion of well. It can also be understood that the second silicon oxide layer is recessed like wells, forming a shape of wells on the upper surface of the transition metal oxide layer. The term "chip matrix" can be divided into single chips that can be assembled into a sequencing chip for testing. The wafer structure contains dozens to thousands of identical single chips (depending on the size of the wafer and chip size), and a non-functional interval is remained between the chips, which is also called the cutting line.

The preparation of the sequencing chip of the present disclosure is not particularly limited. Conventional methods in the existing technologies for preparing sequencing chips from the wafer material can be used according to the differences of the wafer materials used. The difference from existing sequencing chips is that the single chips used are different.

The term "single chip" refers to the one obtained by cutting the "chip matrix" in the present disclosure along the cutting line, and is also called "chip particle".

### Sequencing chip

In the first aspect of the present disclosure, the present disclosure proposes a sequencing chip. According to the embodiment of the present disclosure, the sequencing chip includes: a chip main body, nucleic acids, and a phosphonic acid polymer layer. Wherein, the chip main body includes a plurality of chip particles arranged in a same layer, the chip particles are obtained by cutting a chip matrix along cutting lines of a wafer layer, and the chip matrix includes: a wafer layer having the cutting lines uniformly distributed thereon; a first silicon oxide layer made of silicon oxide and formed on an upper surface of the wafer layer; and a transition metal oxide layer made of a transition metal oxide and formed on an upper surface of the first silicon oxide layer, the nucleic acids are fixed on the transition metal oxide layer; and the phosphonic acid polymer layer is made of a polyphosphonic acid polymer and formed on an upper surface of the transition metal oxide layer.

According to an embodiment of the present disclosure, the polyphosphonic acid polymer is a poly(alkenylphosphonate) or a poly(alkenylphosphonic acid) block copolymer salt.

According to an embodiment of the present disclosure, the polyphosphonic acid polymer is a poly(vinylphosphonate) or a poly(vinyl/propenylphosphonic acid) - poly(vinyl/propenyl carboxylic acid) block copolymer salt, wherein, the poly(vinylphosphonate) has the structure shown in Formula (I),

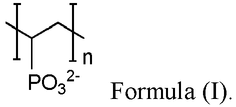

According to an embodiment of the present disclosure, the polyphosphonic acid polymer described in Formula (I) has a molecular weight ranging from, but not limited to, 5 W to 500 W.

According to an embodiment of the present disclosure, the poly(vinylphosphonic acid)-poly(propenylcarboxylic acid) block copolymer salt has the structure shown in Formula (II), 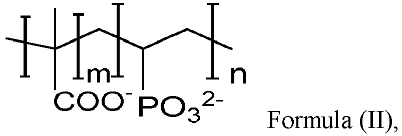 where n is but not limited to an integer from 0 to 76 and m is but not limited to an integer from 380 to 22,000.

According to an embodiment of the present disclosure, the m segments of the polyphosphonic acid polymer shown in Formula (II) have a molecular weight ranging from 0 w to 1 w, but not limited thereto, while the n segments have a molecular weight ranging from 5 w to 500 w, but not limited thereto.

According to an embodiment of the present disclosure, the polyphosphonic acid polymer is sodium poly(vinylphosphonate) or a sodium salt of the poly(vinylphosphonic acid)-poly(propenylcarboxylic acid) block copolymer.

According to an embodiment of the present disclosure, the nucleic acids are DNBs.

According to an embodiment of the present disclosure, the polyphosphonic acid polymer is bonded to at least a part of transition metal oxide molecules in the transition metal oxide layer through chemical bonds;

According to an embodiment of the present disclosure, the chemical bonds are formed by bonding the part of transition metal oxide molecules to phosphonic acid groups of the polyphosphonic acid polymer.

In the sequencing chip according to the embodiment of the present disclosure, a hydrophilic polyphosphonic acid polymer membrane is used to replace the existing protein membrane to fix the DNA nano balls. The polymer film can firmly lie on the surface of the transition metal oxide of the chip main body through the chemical reaction of phosphonic acid and transition metal oxide, thus fixing the DNA nano balls. Due to its macromolecular characteristic, a network structure will be formed when it is adsorbed to the surface, thus ensuring the stability of the DNA nano balls on the chip surface. According to the embodiment of the present disclosure, the sequencing chip is more stable, the sequencing result is more reliable, the data output efficiency of the sequencing chip can be significantly improved, the output of the sequencing chips can be improved, and the sequencing cost can be significantly reduced.

The surface of the chip matrix according to an embodiment of the present disclosure includes two regions, namely, the binding site region of the sequence to be sequenced (especially DNB) (transition metal oxide region, i.e., functional region) and the non-binding site region of the sequence to be sequenced (silicon oxide region, i.e., non-functional region). The inventor found that by taking advantage of the different surface properties of the transition metal oxide region and silicon oxide region on the chip matrix, the sequence to be sequenced can be selectively adsorbed on the transition metal oxide region only by changing the pH of the solution containing the sequence to be sequenced and the surfactant composition. In addition, the transition metal oxide region and the non-functional region can be selectively modified to further enhance the selective adsorption capacity of DNBs on the transition metal oxide region.

According to an embodiment of the present disclosure, the transition metal oxide layer is made of a plurality of unconnected transition metal oxide spots. Transition metal oxide can be discretely distributed on the surface of silicon oxide by conventional methods such as sputtering, electron beam evaporation, or thermal evaporation atomic layer deposition to form a patterned transition metal oxide layer in the shape of "spots". Thus, observed from the surface, the transition metal oxide spots that can be specifically bound to sequencing sequences and silicon oxide regions that are located between the spots and cannot be bound to sequencing sequences are formed on the chip matrix.

According to an embodiment of the present disclosure, the thickness of the transition metal oxide spots ranges from 10 nm to 20 nm, and the thickness of the first silicon oxide layer ranges from 80 nm to 100 nm, preferably 90 nm. Through simulation calculation, the inventor found that the chip matrix in which the thickness of transition metal oxide spots ranges from 10 nm to 20 nm and the thickness of the silicon oxide layer, the first silicon oxide layer ranges from 80 nm to 100 nm, preferably 90 nm, has a higher reflectivity to the light emitted by the DNB sequencing sequence, so that the light signals emitted by the sequences to be sequenced, DNBs, can be captured by the signal detection device as many as possible, which indirectly enhances the signal intensity of DNBs, increases the signal-to-noise ratio, and significantly improves the performance of the finally obtained sequencing chip.

According to an embodiment of the present disclosure, the transition metal oxide spots further have amino groups connected thereon. The inventor found that the amination of transition metal oxide molecules can further improve the specificity of functional regions on the matrix surface of the chip for adsorbing DNBs. Therefore, by adjusting the pH of DNB and the composition of surfactant, the specific adsorption of the functional regions of the chip matrix surface for DNBs is stronger.

According to an embodiment of the present disclosure, the first silicon oxide layer between the plurality of unconnected transition metal oxide spots further has polyethylene glycol connected thereon. Therefore, the non-specific adsorption of DNB to the non-functional regions on the matrix surface of the chip is further reduced.

According to an embodiment of the present disclosure, the chip matrix further includes a second silicon oxide layer.

According to an embodiment of the present disclosure, the transition metal oxide layer is a continuous layer structure, and the second silicon oxide layer is formed by silicon oxide as a plurality of connected wells that are connected to each other on the upper surface of the transition metal oxide layer. It should be noted that the continuous layer structure refers to the transition metal oxide layer fully spread on the upper surface of the first silicon oxide layer. Thus, a patterned pattern of transition metal oxide and silicon oxide that alternate with each other can be obtained by covering the transition metal oxide layer with one or more well-shaped second silicon oxide layers.

According to an embodiment of the present disclosure, the transition metal oxide layer is composed of a plurality of unconnected transition metal oxide spots, and the second silicon oxide layer is formed on the upper surface of the first silicon oxide layer between the plurality of unconnected transition metal oxide spots. Understandably, here the second silicon oxide layer can form a shape of wells with the transition metal oxides spots, wherein, the transition metal oxide is located in the recessed portions of the wells, and the second silicon oxide layer constitutes the bodies of the wells, such that the second silicon oxide layer may be higher than the transition metal oxide layer, or as high as the transition metal oxide layer.

According to an embodiment of the present disclosure, the wafer is a silicon wafer, the second silicon oxide layer has a thickness ranging from 40 nm to 60 nm, preferably 50 nm, the transition metal oxide layer has a thickness ranging from 5 nm to 15 nm, and the first silicon oxide layer has a thickness ranging from 80 nm to 100 nm, preferably 90 nm. Through simulation calculation, the inventor found that when the wafer is a silicon wafer, the thickness of the second silicon oxide layer of the chip matrix forming the well structures ranges from 40 nm to 60 nm, preferably 50 nm, the thickness of the transition metal oxide layer ranges from 5 nm to 15 nm, and the thickness of the first silicon oxide layer ranges from 80 nm to 100 nm, preferably 90 nm, it has a higher reflectivity to the light emitted by the DNB to be sequenced, so that the light signals emitted by the sequences to be sequenced, especially DNBs, can be captured by the signal detection device as many as possible, which indirectly enhances the signal intensity of the DNB to be sequenced, increases the signal-to-noise ratio, and significantly improves the performance of the finally obtained sequencing chip.

According to an embodiment of the present disclosure, the wafer is a quartz wafer, the thickness of the second silicon oxide layer ranges from 100 nm to 200 nm, the thickness of the transition metal oxide layer ranges from 10 nm to 20 nm, and the thickness of the first silicon oxide layer ranges from 80 nm to 100 nm, preferably 90 nm. Through simulation calculation, the inventor found that when the wafer is a quartz wafer, the thickness of the second silicon oxide layer of the chip matrix forming the well structures ranges from 100 nm to 200 nm, the thickness of the transition metal oxide layer ranges from 10 nm to 20 nm, and the thickness of the first silicon oxide layer ranges from 80 nm to 100 nm, preferably 90 nm, it has a higher reflectivity to the light emitted by the sequence to be sequenced, especially DNB, so that the light signals emitted by the DNBs to be sequenced, can be captured by the signal detection device as many as possible, which indirectly enhances the signal intensity of the DNBs to be sequenced, increases the signal-to-noise ratio, and significantly improves the performance of the finally obtained sequencing chip. In addition, when the thickness of the second silicon oxide layer ranges from 100 nm to 200 nm, the sequencing chip finally formed not only ensures that the well structures have an appropriate depth to load the DNB to be sequenced, but also enables the camera to capture fluorescence signal with relatively high intensity.

According to an embodiment of the present disclosure, the transition metal oxide layer or the transition metal oxide spots at the recessed portions of wells of the second silicon oxide layer are further connected with amino groups. The inventor found that the amination of the transition metal oxide molecules can further improve the specificity of the functional regions on the chip matrix surface in adsorbing the sequences to be sequenced. Therefore, by adjusting the pH of the sequences to be sequenced and surfactant composition, the specific adsorption of the sequences to be sequenced on the functional regions on the chip matrix surface can be realized.

According to an embodiment of the present disclosure, the second silicon oxide layer is further connected with polyethylene glycol. As a result, the non-specific adsorption of the non-functional regions on the chip surface for DNBs is further reduced.

According to an embodiment of the present disclosure, the amino groups are bonded to at least a part of transition metal oxide molecules in the transition metal oxide layer through chemical bonds. Wherein, "chemical bond" refers to a transition metal-O-P bond (such as Zr-O-P bond, Ti-O-P bond, Ta-O-P bond). As a result, the amino groups and the transition metal oxide can bind with each other closely.

According to an embodiment of the present disclosure, the chemical bond is formed by bonding a transition metal oxide molecule to a phosphonic acid group of an aminophosphonic acid compound. Based on the fact that a phosphonic acid group does not react with silicon oxide later, but reacts specifically with transition metal oxide molecules, the inventor uses the aminophosphonic acid compound to specially introduce amino groups on the transition metal oxide molecules.

According to an embodiment of the present disclosure, the polyethylene glycol is provided by at least one of polyethyleneimine-polyethylene glycol and a silane coupling agent containing polyethylene glycol. Therefore, the non-specific adsorption of the non-functional regions on the chip matrix surface for DNBs is further reduced.

According to an embodiment of the present disclosure, the polyethylene glycol is provided by polyethyleneimine-polyethylene glycol, and the polyethyleneimine-polyethylene glycol is electrostatically adsorbed on the surface of the first silicon oxide layer or the surface of the second silicon oxide layer.

According to an embodiment of the present disclosure, the polyethylene glycol is provided by a silane coupling agent containing polyethylene glycol, and the silane coupling agent containing polyethylene glycol is connected to the first silicon oxide layer or the second silicon oxide layer through an -Si-O-Si- chain.

It should be noted that the material of the wafer according to the embodiment of the present disclosure is not limited. According to a specific embodiment of the present disclosure, the wafer includes at least one selected from a silicon wafer, a quartz wafer, a glass wafer, or a CMOS wafer.

According to an embodiment of the present disclosure, the transition metal oxide include at least one selected from titanium dioxide, zirconium dioxide, tantalum pentoxide, niobium hexoxide, or hafnium dioxide.

According to an embodiment of the present disclosure, the transition metal oxide include at least one selected from titanium dioxide, zirconium dioxide, or tantalum pentoxide.

The sequencing chip according to the present disclosure does not need a monomolecular layer on the surface of the structure, or surface modification can be performed after the chip preparation process is completed, thus the sequencing chip described in the present disclosure has stable characteristics, which can withstand physical contacts such as scratches without affecting the sequencing chip performance, and has resistance to high temperature and resistance to chemical corrosion. As a result, the chip can withstand processing and assembly with more stringent conditions but higher efficiency, and is less susceptible to damage during packaging, transportation, and preparation for use. Therefore, it improves the yield of sequencing chips, and increases the efficiency of using sequencing chips to produce data, thus reducing the cost.

### Method of preparing sequencing chip

In a second aspect of the present disclosure, the present disclosure provides a method for preparing a sequencing chip. According to an embodiment of the present disclosure, the method includes: 1) performing surface modification on the wafer layer to obtain the chip matrix, wherein the surface modification includes processing a surface of the wafer layer with a transition metal oxide to form the transition metal oxide layer, the wafer layer has a first silicon oxide layer made of silicon oxide on an upper surface thereof, the transition metal layer being formed on the upper surface of the first silicon oxide layer, and the wafer layer having the cutting lines uniformly distributed thereon; 2) cutting the chip matrix along the cutting lines of the wafer layer to obtain chip particles; 3) obtaining the chip main body by assembling the chip particles; 4) fixing DNA nano balls on the chip main body; and 5) incubating the chip main body fixed with the DNA nano balls in a buffer solution of the polyphosphonic acid polymer to obtain the sequencing chip. The method according to the embodiment of the present disclosure is simple in operations and has a high yield of the prepared sequencing chip.

According to an embodiment of the present disclosure, in step 5), the buffer solution is a PBS buffer solution.

According to an embodiment of the present disclosure, in step 5), the polyphosphonic acid polymer has a molecular weight ranging from 5 W to 510 W.

According to an embodiment of the present disclosure, in step 5), a concentration of the polyphosphonic acid polymer in the buffer ranges from 1.5 mg/mL to 2.5 mg/mL, preferably 2 mg/mL.

According to an embodiment of the present disclosure, in step 5), the incubating is performed at a room temperature for 8 min to 12 min, for example 8 min, 9 min, 10 min, 11 min, and 12 min.

According to an embodiment of the present disclosure, in step 1), the first silicon oxide layer is formed in advance on the upper surface of the wafer layer by low-temperature plasma chemical vapor deposition, plasma-enhanced chemical vapor deposition, sputtering, or atomic layer deposition. It should be noted that the method for forming the first silicon oxide layer on the wafer surface is not limited and can be carried out through conventional semiconductor process techniques, such as low-temperature plasma chemical vapor deposition, plasma-enhanced chemical vapor deposition, sputtering, atomic layer deposition, etc.

According to an embodiment of the present disclosure, in step 1), the surface modification on the wafer layer is achieved by thin film deposition, photoetching, or etching in order to form a continuous transition metal oxide layer or a transition metal oxide layer arranged as spots. According to a specific embodiment of the present disclosure, a patterned transition metal oxide layer is formed on the silicon oxide layer, the transition metal oxide can be titanium dioxide, zirconium dioxide, tantalum pentoxide, niobium hexoxide, hafnium dioxide, or any combination thereof, the transition metal oxide layer is discretely distributed on the silicon oxide layer, forming a specific array pattern (that is, an array of transition metal oxide spots and specially designed graphics or lines, for calibration in later sequencing optics), with the same pattern arrangement on every single chip. This patterned layer can be realized by conventional semiconductor technology, such as thin film deposition, photoetching, and etching, that is, a layer of transition metal oxide layer covering the whole wafer is formed on the silicon oxide layer by sputtering, electron beam evaporation, thermal evaporation atomic layer deposition or other thin film deposition technologies; then a hard mask material layer corresponding to the required patterned layer is formed on the metal oxide layer by thin film deposition, photoetching, or etching; at last, the pattern of the hard mask material layer is reproduced onto the transition metal oxide layer by etching process, to form a patterned transition metal oxide layer, namely discretely distributed transition metal oxide orderly arranged in a shape of "spots" on the silicon oxide layer, and regions with no transition metal oxide "spot" exposes the silicon oxide layer, wherein the size of the transition metal oxide region in a shape of a "spot" is the same as or slightly smaller than that of DNB, so that one "spot" only adsorbs one DNB.

According to an embodiment of the present disclosure, the transition metal oxide layer is a continuous layer structure, and in step 1), the method further includes forming a second silicon oxide layer made of silicon oxide in an arrangement of continuous wells on the upper surface of the transition metal oxide layer. Wherein, the formation here is mainly realized by atomic layer deposition. According to a specific embodiment of the present disclosure, a first silicon oxide layer is formed on the wafer, and then a transition metal oxide layer is formed on the first silicon oxide layer, and then a discretely arranged array of "well" structures is formed on the transition metal oxide layer by photoetching and etching techniques in the conventional semiconductor technologies. The bottom of the "well" structure is the exposed transition metal oxide layer, and the periphery of the "well" structure is the silicon oxide layer higher than the transition metal oxide layer. The size of the "well" is the same as or slightly smaller than the size of the DNB, so that each "well" structure only bonds with one DNB.

According to an embodiment of the present disclosure, the transition metal oxide layer is arranged as spots, and in step 1), the method further includes depositing silicon oxide between the spots of the transition metal oxide layer to form a second silicon oxide layer. Wherein, the depositing here is mainly achieved by atomic layer deposition. According to a specific embodiment of the present disclosure, a first silicon oxide layer is formed on the wafer, and then a discretely arranged array of "well" structures is formed on the silicon oxide layer by photoetching and etching techniques in the conventional semiconductor technologies. The bottom of the "well" structure is the exposed transition metal oxide layer, and the pheripery of the "well" structure is the silicon oxide layer as high as or higher than the transition metal oxide layer. The size of the "well" is the same as or slightly smaller than the size of the DNB, so that each "well" structure only bonds with one DNB.

According to an embodiment of the present disclosure, the method further includes, subsequent to step 3) and prior to step 4), preforming amination treatment on the transition metal oxide. Therefore, amino groups can be introduced into the functional regions of the chip matrix to further improve the specific adsorption capacity of the functional regions for the sequences to be sequenced, especially DNBs.

According to an embodiment of the present disclosure, the amination treatment is achieved by reacting the transition metal oxide with an aminophosphonic acid compound. Thus, the aminophosphonic acid compound can form transition metal-O-P bonds (such as Zr-O-P bond, Ti-O-P bond, Ta-O-P bond) with the transition metal oxide. Furthermore, the amino groups can be introduced into the functional regions of the chip matrix to further improve the specific adsorption capacity of the functional regions for the sequences to be sequenced, especially DNBs.

According to an embodiment of the present disclosure, the method further includes, subsequent to step 3) and prior to step 4), performing surface modification on the first silicon oxide layer or the second silicon oxide layer to introduce polyethylene glycol on the first silicon oxide layer or the second silicon oxide layer. Thus, the adsorption capacity of the non-functional regions of the chip matrix for DNB sequencing sequences can be further reduced.

According to an embodiment of the present disclosure, the polyethylene glycol is provided by at least one of polyethylenemine-polyethylene glycol and a silane coupling agent containing polyethylene glycol.

According to an embodiment of the present disclosure, the polyethylene glycol is provided by polyethyleneimine-polyethylene glycol, and the surface modification is peformed by electrostatic adsorption of the polyethyleneimine-polyethylene glycol to a surface of the first silicon oxide layer or a surface of the second silicon oxide layer. Thus, polyethylene glycol can be introduced into the non-functional regions of the chip matrix.

According to an embodiment of the present disclosure, the polyethylene glycol is provided by a silane coupling agent containing polyethylene glycol, and the surface modification is performed by condensation reaction of the silane coupling agent containing polyethylene glycol with hydroxyl groups of the first silicon oxide layer or the second silicon oxide layer. The hydroxyl groups are provided by Si-OH formed after the first or second silicon oxide layer is ionized and then adsorbs hydroxide ions in water. Thus, polyethylene glycol can be introduced into the non-functional regions of the chip matrix.

According to an embodiment of the present disclosure, in step 2), the cutting is performed by a semiconductor wafer cutting method.

According to an embodiment of the present disclosure, in step 3), the assembling includes placing the chip particle in a support frame having a liquid inflow-outflow opening, and attacting the chip particle to the support frame with a glue or an adhesive, a fluid channel being formed between the support frame and the chip particle.

According to an embodiment of the present disclosure, the wafer is a silicon wafer, and the assembling includes: attaching the chip particle to the support frame with an upper surface of the chip particle facing upward, and providing a cover glass on the upper surface of the chip particle to obtain the chip main body.

According to an embodiment of the present disclosure, the wafer is a quartz wafer or a glass wafer, and the assembling includes: attaching the chip particle to the support frame with a lower surface of the chip particle facing upward to obtain the chip main body.

According to an embodiment of the present disclosure, the wafer is a CMOS wafer, and the assembling includes: attaching a lower surface of the chip particle to a substrate (i.e.g, a photosensitive element), bonding the chip particle to the substrate by a lead wire to obtain the chip main body, the lead wire being configured to transmit an electrical signal on the chip particle to the substrate.

According to an embodiment of the present disclosure, the substrate is in a form including but not limited to LGA, CLCC, PLCC, etc.

According to an embodiment of the present disclosure, the metal wires used for lead wire bonding include but are not limited to gold wires and aluminum wires, etc.

### Sequencing methods

In a third aspect of the present disclosure, the present disclosure provides a sequencing method. According to an embodiment of the present disclosure, the method includes performing sequencing by using the sequencing chip as previously defined or prepared by the method previously defined. According to an embodiment of the present disclosure, the transition metal oxide layer of the sequencing chip is fixed with DNBs. ADNB can be considered as a point light source, light emitted from which is captured by a camera or CMOS image sensor and then sequenced. According to the method in an embodiment of the present disclosure, multiple cycle sequencing can be carried out, and the sequencing results are of high accuracy and low cost.

Examples of the present disclosure are described in detail below.

### Example 1: a method for preparing a sequencing chip main body with "spot" structures on a silicon or quartz wafer

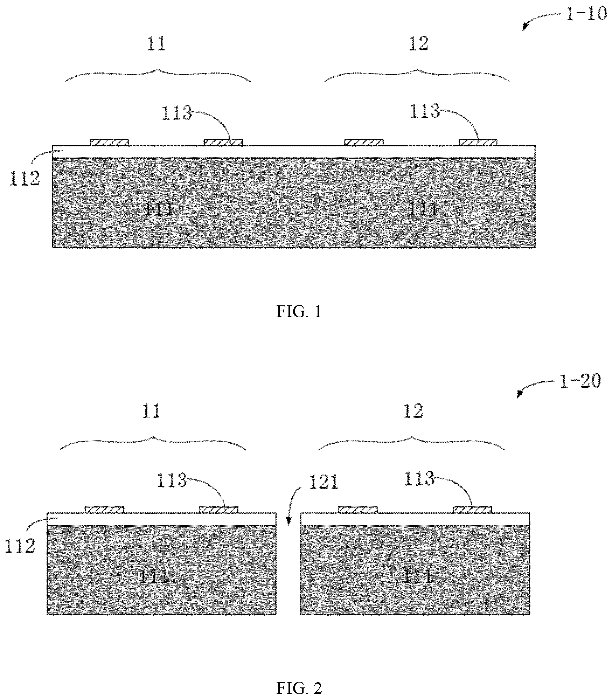

Refering to FIGS. 1 to 5, in this example, a method for preparing a sequencing chip having transition metal oxide "spot" structures on a silicon or quartz wafer was proposed, and sectional views of the processes for steps of the method are shown. The method can be done on a raw wafer that does not contain any internal circuits or structures. The diagrams of the present disclosure schematically show only two regions 11 and 12 on the wafer, those skilled in the art should be aware that a plurality of single chips with the same structure can be formed on the wafer (depending on the size of the wafer and the size of the chip, the number of chips can vary from several dozens to thousands), every single chip can form a sequencing chip main body.

FIG. 1 shows a sectional view of a wafer 1-10 in which a patterned layer, in which DNB binding site regions (transition metal oxide layers, i.e., functional regions) and DNB non-binding site regions (silicon oxide layers, i.e., non-functional regions) are present alternately, was formed on a raw wafer. Firstly, a wafer substrate structure 111 in FIG. 1 was provided. The material thereof may be silicon or quartz, but those skilled in the field should be aware that the present disclosure does not intend to limit the substrate material to silicon or quartz, and any other suitable semiconductor wafer material may also be used in the present disclosure. A silicon oxide layer 112 was then formed on the wafer 111 by conventional semiconductor technology, such as low-temperature plasma chemical vapor deposition, plasma-enhanced chemical vapor deposition, sputtering, and atomic layer deposition, etc. Then, a patterned transition metal oxide layer was formed on the silicon oxide layer. The transition metal oxide could be titanium dioxide, zirconium dioxide, tantalum pentoxide, niobium hexaoxide, hafnium dioxide, or any combination thereof. Such transition metal oxide layer was discretely distributed on the silicon oxide layer and formed a particular array pattern, and every single chip (such as single chips 11 and 12 in FIG. 1) had the same pattern arrangement. This patterned layer can be realized by conventional semiconductor technology, such as thin film deposition, photoetching, and etching, that is, a transition metal oxide layer (not shown) covering the entire wafer was formed on the silicon oxide layer by sputtering, electron beam evaporation, thermal evaporation atomic layer deposition, or other film deposition technologies; then, a hard mask material layer (not shown) corresponding to the required patterned layer was formed on the metal oxide layer by thin film deposition, photoetching, or etching. At last, the pattern of the hard mask material layer was reproduced onto the transition metal oxide layer by etching, to form a patterned transition metal oxide layer shown as structure 113 in FIG.1. In the patterned layer shown as structure 113, the discretely arranged transition metal oxide was orderly arranged in a shape of "spots" on the silicon oxide layer, regions with no transition metal oxide "spots" exposed the silicon oxide layer, and the size of the "spot"-like transition metal oxide region was the same as or slightly smaller than that of DNB so that one "spot" only adsorbed one DNB. It should be noted that the process steps required to form such a patterned layer were described here, but any process to achieve such a patterned layer should be included in the present disclosure. The wafer structure 1-10 in FIG. 1 may contain dozens to thousands of identical chips (depending on wafer size and chip size), and a very narrow non-functional interval was retained between chip and chip. Such an interval was also called the cutting line. A cutting knife could cut the wafer into a plurality of single chips without damaging the effective structure regions of the chips.

FIG. 2 shows a sectional view of multiple single wafer structures 1-20 formed by cutting the wafer 1-10 having a patterned transition metal oxide layer. In FIG. 2, the wafer had been cut into single chips separated by the cutting slot 121. The single chips 11 and 12 formed after the cutting process were schematically shown in FIG. 2.

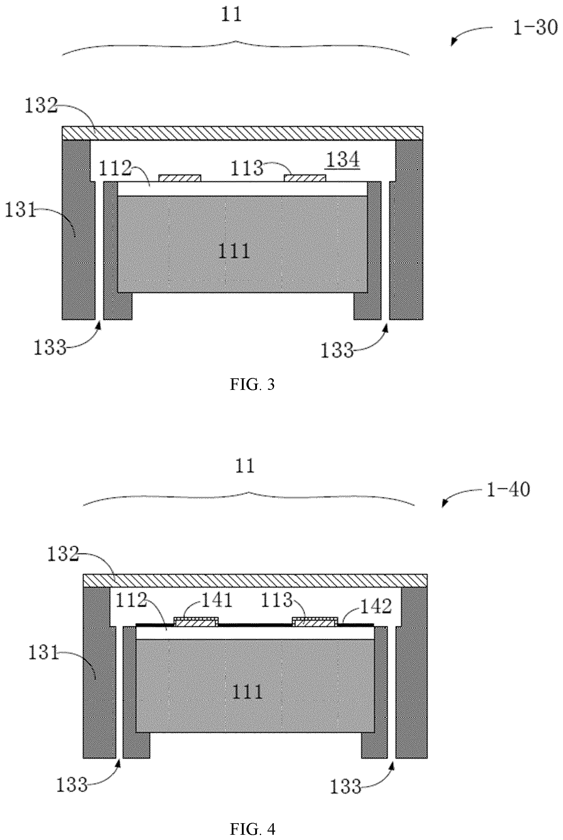

FIG. 3 shows a sectional view of a sequencing chip 1-30 formed by assembling a single chip. A single chip with a patterned surface layer 131 was first installed into a frame structure 131 containing liquid inflow-outflow opening 133, then a cover glass 132 that had undergone hydrophobic treatment was attached to the frame structure 131, and a fluid channel 134 was formed between the cover glass 132 and the chip with a patterned surface 113. A liquid could enter or exit from the fluid channel 134 from the inflow-outflow opening 133. In this figure, the chip was assembled into the frame 131 and the cover glass 132 was attached to the frame 131 by means of adhesives. The adhesive was used to fix each part, and any suitable adhesive can be used in the present disclosure. This figure schematically depicted the structure of the assembled sequencing chip, namely including the frame providing the fluid inflow-outflow opening, and the cover glass forming the fluid channel with the frame. But those skilled in the art should be aware that frames and cover glasses made of any suitable materials, and any frame and cover glass structures which can provide the fluid inflow-outflow opening and fluid channel should be included in the present disclosure.

FIG. 4 shows a sequencing chip 1-40 formed after surface functional modification of the sequencing chip 1-30. Wherein the liquid used for surface modification can enter the fluid channel through the liquid inflow-outflow opening, and contact with the transition metal oxide region and silicon oxide region to functionally modify the surfaces thereof, making them obtain a function of adsorbing DNB (namely the DNB binding site, transition metal oxide region, functional region) and a function of repelling DNB (namely the DNB non-binding site, silicon oxide region, non-functional region) respectively. The surface modification process included: 1) forming a polyethylene glycol molecule layer 142 on the surface of the silicon oxide layer. The polyethylene glycol molecule included at least one selected from polyethyleneimine-polyethylene glycol and a silane coupling agent containing polyethylene glycol, thus, the non-specific adsorption of the nonfunctional region of the chip surface for DNBs was further reduced; or the polyethylene glycol molecule was polyethyleneimine-polyethylene glycol, and one end of the polyethylene glycol molecule was connected to the silicon oxide layer 112 by electrostatic adsorption. Polyethyleneimine-polyethylene glycol can be self-assembled on the silicon oxide layer 112, and then adsorbed on the silicon oxide layer 112 by electrostatic action. Or the polyethylene glycol molecule was a silane coupling agent containing polyethylene glycol, the plurality of polyethylene glycol molecules each had one end connected to the silicon oxide layer 112 by a -Si-O-Si- chain, and a condensation reaction occurred between the silane coupling agent containing polyethylene glycol and the hydroxyl group on the surface of the silicon oxide layer 112 to form the -Si-O-Si- chain. 2) forming a plurality of amino groups 141 on the transition metal oxide layer. A plurality of amino groups was connected to at least part of the plurality of transition metal oxide molecules, and the plurality of amino groups was not connected to the silicon oxide layer. The inventor found that the amination of transition metal oxide molecules can further improve the specificity of the functional regions of the chip surface for adsorbing DNBs. Therefore, the specific adsorption of the functional regions of the chip surface for DNBs can be realized by adjusting the pH of DNBs and the composition of surfactants. The plurality of amino groups was provided by aminophosphonic acid compounds. The inventor found that the phosphonic acid group does not react with the silicon oxide layer, but specifically reacts with the transition metal oxide molecule, aminophosphonic acid compounds can be used to specifically introduce amino groups into the transition metal oxide molecules; or the plurality of amino groups was bonded to at least part of the plurality of transition metal oxide molecules by chemical bonds. As mentioned above, the inventor used the phosphonic groups to react with transition metal oxide molecules without reacting with the silicon oxide layer, and used aminophosphonic acid compounds to specifically introduce amino groups into transition metal oxide molecules. Wherein, phosphonic acid groups can form corresponding chemical bonds with transition metal oxide molecules. The plurality of amino groups were connected to transition metal oxide molecules by the chemical bonds formed by the transition metal oxide molecules and the phosphonic acid groups. The chemical bond was formed by a connection between transition metal and a phosphonic acid group of an aminophosphonic acid compound. A phosphonic acid group in aminophosphonic acid compounds can form a corresponding transition metal-O-P bond (such as Zr-O-P bond, Ti-O-P bond, Ta-O-P bond) with the transition metal oxide molecule, so the plurality of amino groups is connected to transition metal oxide molecules through transition metal-O-P bonds formed by phosphonic acid groups and the transition metal oxide molecules.

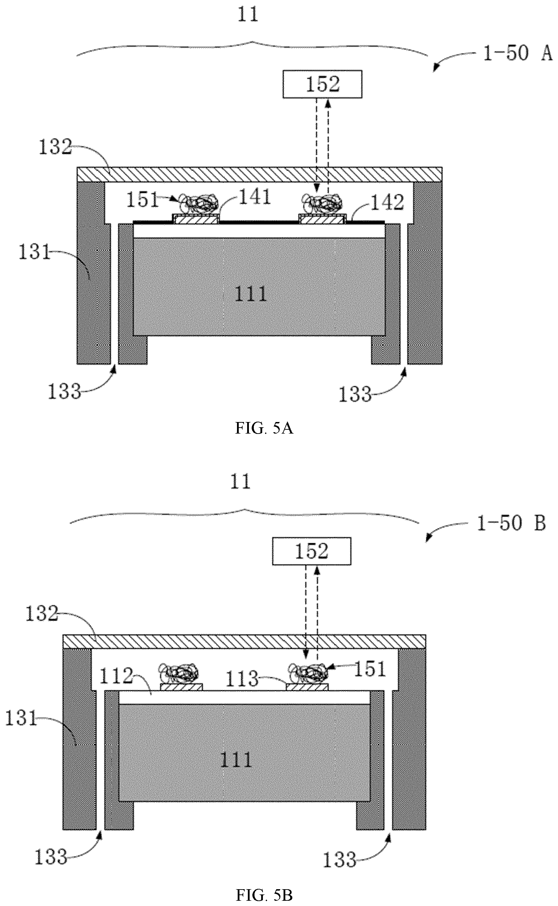

FIG. 5A shows a sectional view of a sequencing chip 1-50A containing a DNB array formed after loading of DNBs on the functionally modified sequencing chip main body 1-40 in FIG. 4. A DNB reagent entered the fluid channel through the liquid inflow-outflow opening on the sequencing chip, then DNBs selectively bonded with DNB binding sites (amino groups-modified transition metal oxide region, namely the functional regions), without bonding with the DNB non-binding sites (polyethylene glycol-modified silicon oxide layer), thus forming the DNB nanoarray. Light source and camera 152 were also shown in FIG.5A. DNBs bonded with fluorescent markers may emit light with a specific wavelength or energy under excitation of the light source of a specific wavelength or energy, and the light may be collected by the camera. The base arrangement on DNB could be identified by analyzing the light signal collected by the camera.

FIG. 5B shows another more concise DNB loading method. In FIG. 5B, the sequencing chip 1-30 of FIG. 3 can be loaded with DNBs without any surface modification. This step requires to optimize the pH of DNB reagent and surfactant composition, so that DNBs could be selectively adsorbed on DNB binding sites (transition metal oxide layer, i.e., functional region) and repelled by DNB non-binding sites (silicon oxide layer, i.e., non-functional region) without surface functional modification. It should be noted that the method of surface functional modification followed by DNB loading as shown in FIG. 4 and 5A would result in a better selective adsorption effect of DNBs on the pattered surface.

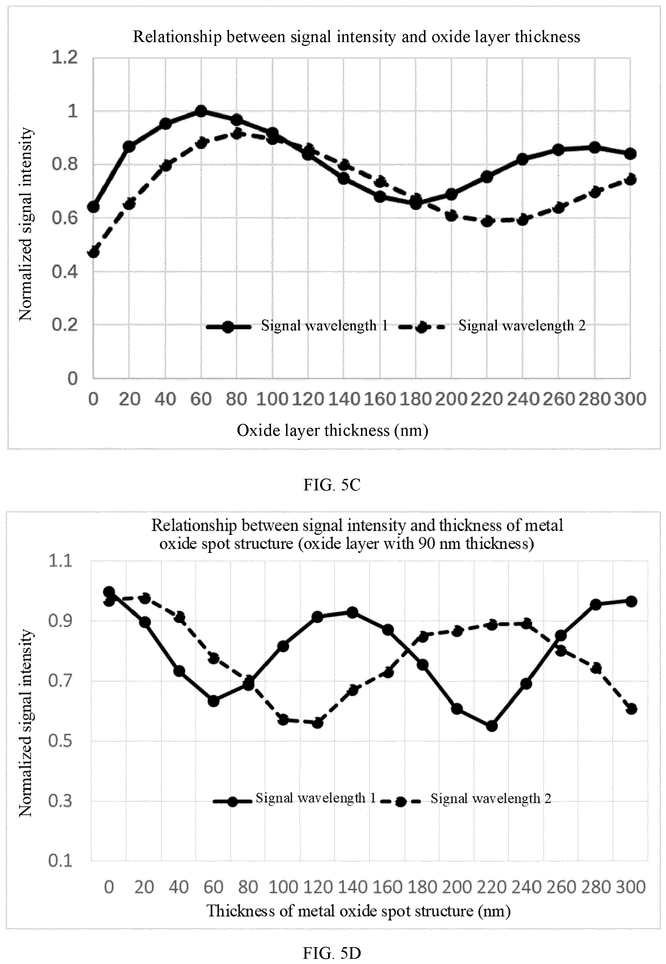

The results are shown in FIG. 5A and 5B. DNB sample 151 was loaded on transition metal oxide "spot" 113, and camera 152 was placed above the DNB sample to collect the light signal emitted by the DNB sample. DNB sample could be considered as a point light source, and the light emitted upward by the DNB sample was directly collected by the camera, while the light emitted downward by the DNB sample was partially reflected by the transition metal oxide layer and silicon oxide layer and collected by the camera, and the other part of the downward emitted light passed through the transition metal oxide layer and silicon oxide layer and entered the silicon substrate. The inventor obtained an optimized thickness of the transition metal oxide layer and silicon oxide layer by optical simulation calculation. At this thickness, the transition metal oxide layer and silicon oxide layer had the maximum reflection and the minimum transmission to the light signal emitted by DNB, so that the light signals emitted from the DNB were transmitted upward and collected by the camera as many as possible, and the fluorescence signal captured by the camera had the maximum intensity. During the simulation calculation, in order to determine an optimal silicon oxide layer thickness, the fluorescence signal intensities corresponding to different thicknesses of the silicon oxide layer were calculated in the absence of a transition metal oxide layer. The simulation results are shown in FIG. 5C. When the thickness of the silicon oxide layer was about 90 nm, the reflectivity of the four wavelengths of light was relatively high.

When the thickness of the silicon oxide layer was about 90 nm, the silicon oxide layer had the best reflection effect on the light signal emitted by the DNB sample, that is, the intensity of the fluorescence signal captured by the camera was the strongest. Then, when the thickness of the silicon oxide layer was 90 nm, the relationship between the change of transition metal oxide layer thickness and fluorescence signal intensity was calculated and silmulated, and the results are shown in FIG. 5D. When the thickness of the transition metal oxide layer was less than 40 nm, the intensity of the fluorescence signal captured by the camera gradually reduced with the increase of the thickness of the transition metal oxide layer. Therefore, when the thickness of the transition metal oxide layer was about 10 to 20 nm, the transition metal oxide layer had good mechanical reliability and maximum reflectivity, and the intensity of fluorescence signal captured by the camera was the highest, as shown in FIG. 5D.

### Example 2: a method for preparing a sequencing chip main body having transition metal oxide "well" structures on a silicon or quartz wafer

Reference to FIGS. 6 to 11, in this example, a method for preparing a sequencing chip main body having transition metal oxide "well" structures on a silicon or quartz wafer is proposed, and sectional views of the processes for steps of the method are shown. The method can be done on a raw wafer that does not contain any internal circuits or structures. The diagrams of the present disclosure schematically show only two regions 21 and 22 on the wafer, those skilled in the art should be aware that a plurality of single chips with the same structure can be formed on the wafer (depending on the size of the wafer and the size of the chip, the number of chips can vary from several dozens to thousands), and every single chip can form a sequencing chip.

FIG. 6 shows a sectional view of a wafer structure 2-10 formed after a silicon oxide layer and a transition metal oxide layer were formed on the raw wafer. A wafer substrate 211 was provided first, which may be made of, but not limited to, a silicon or quartz material, and any suitable semiconductor wafer may be applied to the present disclosure. A silicon oxide layer 212 was then formed on the wafer 211 by a process similar to that described in FIG. 1 of Example 1. A transition metal oxide layer 213 was then formed on the silicon oxide layer. The transition metal oxide could be titanium dioxide, zirconium dioxide, tantalum pentoxide, niobium hexoxide, hafnium oxide, or any combination thereof, and the formation method was similar to that in FIG 1 of Example 1.

FIG. 7 shows a sectional view of the wafer 2-20 formed after the patterned silicon oxide layer having "well" structures was formed on the wafer 2-10 with a transition metal oxide layer in FIG. 6. In FIG.7, a silicon oxide layer 221 was first formed on the wafer 2-10 in FIG. 6, and then a discretely arranged array of "well" structures 222 was formed on the silicon oxide layer 221 by conventional semiconductor processes such as photoetching and etching techniques. The bottom of the "well" structure was the exposed transition metal oxide layer, and the periphery of the "well" structure was the silicon oxide layer 221 that was higher than the transition metal oxide layer. The size of the "well" was the same as or slightly smaller than the size of the DNB, so that each "well" structure only bonded with one DNB.

FIG. 8 shows a sectional view of multiple single chips 2-30 formed by cutting the wafer structure 2-20 in FIG. 7. The cutting process similar to that in FIG. 2 of Example 1 was used in this figure. The wafer 2-20 in FIG. 7 was cut into single chips 21 and 22 separated by a cutting slot 231.

FIG. 9 shows a sectional view of a sequencing chip 2-40 formed by assembling a single chip. The assembling process was similar to that in FIG. 3 of Example 1, including a frame 241 with a liquid inflow-outflow opening 243 and a cover glass 242 attached to the frame. A fluid channel was formed between the cover glass and the single chip containing an array of "well" structures.

FIG. 10 shows a sectional view of a sequencing chip 2-50 formed after surface functional modification of the sequencing chip 2-40 in FIG. 9. Processing steps of surface functional modification in this figure were similar to those in FIG.4 of Example 1, finally forming the amino group-modified DNB binding site region (namely the functional region) on the exposed bottom of the "well" structure on the transition metal oxide layer 213, and forming a polyethylene glycol molecule layer on the surface of the silicon oxide layer higher than the transition metal oxide layer.

FIG. 11A shows a sectional view of a sequencing chip 2-60A with a DNB array formed after loading of DNBs on the sequencing chip 2-50 as shown in FIG. 10. As shown in this figure, DNBs were loaded in the array of "well" structures of the surface functionally modified sequencing chip 2-60A, which would enable DNB to withstand the scouring of liquid with a higher flow rate and improve the sequencing speed of the sequencing chip. The excitation light source and camera structure 262 were also shown in this figure, which can provide excitation light of specific wavelength and energy, and collect light signals of specific wavelength and energy emitted by DNBs labeled with fluorescent markers, for identification of base arrangement on DNB.

FIG. 11B shows another more concise DNB loading method. The sequencing chip 2-40 in FIG. 9 can be loaded with DNBs to form the sequencing chip 2-60B without any surface modification. This step requires to optimize the pH of DNB reagent and surfactant composition, so that DNBs could be selectively adsorbed on DNB binding sites (transition metal oxide layer, i.e., functional region) and repelled by DNB non-binding sites (silicon oxide layer, i.e., non-functional region) without any surface functional modification. The inventor found that the method of surface functional modification followed by DNB loading as shown in FIG. 10 and FIG. 11A would result in a better selective adsorption effect of DNBs on the pattered surface.

The inventor also optimized the thickness of the silicon oxide layer and transition metal oxide layer by optical simulation calculation. The transition metal oxide layer in this example was a layer of film structure, the layer under the transition metal oxide layer was the first silicon oxide layer, and the layer above the transition metal oxide layer was the second silicon oxide layer with the array of "well" structures. According to the simulation results in Example 1 above, the inventor learned that when the thickness of the transition metal oxide layer varied from 0 nm to 40 nm, the reflectivity of the film decreased gradually and the intensity of the fluorescence signal collected by the camera decreased gradually with the increase of the thickness of the transition metal oxide layer. Therefore, first of all, the relationship between fluorescence signal intensity and the thickness of the second silicon oxide layer with an array of "well" structures was simulated under the conditions that the thickness of the first silicon oxide layer was 90 nm and the thickness of the transition metal oxide layer was 0 nm, 10 nm, and 20 nm respectively. Simulation results are shown in FIG. 11C, showing that the fluorescence signal intensity gradually weakened and the reflectivity of the film to the light signal decreased with the increase of the thickness of the second silicon oxide layer. Therefore, in order to make the "well" structure deep enough to load DNB, the inventors selected the thickness of the second silicon oxide layer as about 50 nm.

Provided that the thickness of the first silicon oxide layer was 90 nm and the thickness of the second silicon oxide layer was 50 nm, the corresponding relationship between different transition metal oxide layer thicknesses and fluorescence signal intensities was simulated. Simulation results are shown in FIG. 11D, and the changing trend was similar to that in example 1, that is, when the thickness of the transition metal oxide layer was less than 40 nm, the reflectivity decreased and the fluorescence signal intensity gradually weakened with the increase of the thickness of transition metal oxide layer. Therefore, the inventor believed that the transition metal oxide layer thickness of about 5 to 15 nm was optimal, in this case, fluorescence signals of different wavelengths emiited by the four bases all had relatively high intensities. As shown, the inventor believed that in this example, when the thickness of the first silicon oxide layer was 90 nm, the thickness of the transition metal oxide layer was 5 nm to 15 nm, and the thickness of the second silicon oxide layer was 50 nm, the reflectivity of the film on the sequencing chip was larger, and the intensity of the fluorescence signal that can be captured by the camera was relatively higher.

### Example 3: Another method for preparing a sequencing chip main body having transition metal oxide well" structures on a silicon or quartz wafer

As shown in FIGS. 12 to 17, in this example, sectional views of the processes for steps of another method for preparing a sequencing chip main body having transition metal oxide well" structures on a silicon or quartz wafer were provided. The difference between this method and that of Example 2 was that the patterned transition metal oxide layer 313 was formed on a wafer 311 containing a silicon oxide layer 312 at first in this method, while the transition metal oxide layer 313 was formed on the whole wafer in the method of Example 2, as shown in FIG. 12 and FIG. 6 by comparing.

FIG. 12 shows a sectional view of a wafer structure 3-10 with a patterned transition metal oxide layer 313, whose formation process was similar to that in FIG. 1 of Example 1.

FIG. 13 shows a sectional view of a wafer structure 3-20 formed after a silicon oxide layer 321 with patterned "well" structures 322 was formed on the wafer 3-10 with a patterned transition metal oxide layer shown in FIG. 12. The formation process thereof was similar to that in FIG. 7 of Example 2, wherein the "well" structures 322 on the silicon oxide layer corresponded to the patterned "spot"-like transition metal oxide layers 313 one to one, and eventually, the silicon oxide layer would be higher than the transition metal oxide layer, forming an array of patterned "well" structures on the wafer surface. The bottom of the "well" structure was the exposed transition metal oxide layer 313.

FIG. 14 shows a sectional view of multiple single chips 3-30 separated by a cutting slot 331 formed by cutting the wafer structure 3-20 shown in FIG. 13. The cutting process was similar to that in FIG. 8 of Example 2.

FIG. 15 shows a sectional view of a sequencing chip 3-40 formed by assembling the single chip 3-30 shown in FIG. 14. The assembling process was similar to that in FIG 9 of Example 2.

FIG. 16 shows a sequencing chip 3-50 formed after surface functional modification of the sequencing chip 3-40 shown in FIG. 15. The surface functional treatment process was similar to that in FIG. 10 of Example 2.

FIG. 17A shows a sectional view of a sequencing chip 3-60A with a DNB array formed after loading of DNBs on the sequencing chip 3-50 that had undergone surface functional treatment as shown in FIG. 16. The DNB loading procedure was similar to that in FIG. 11A of Example 2.

FIG. 17B shows another more concise DNB loading method that does not require surface functional modification, which was similar to that in FIG. 11B of Example 2 above.

In this example, the transition metal oxide layer was structured in an array of "spots" formed on the first silicon oxide layer, while a second silicon oxide layer with an array of "well" structures was formed above the transition metal oxide layer. The "well" structure of the silicon oxide layer corresponded to the "spot" of the transition metal oxide layer. In the above example 1, it was obtained through simulation calculation that when the thickness of the first silicon oxide layer was 90 nm and the thickness of the transition metal oxide layer was about 10 to 20 nm, the reflectivity was optimal and the fluorescence signal intensity was maximized. On this basis, provided that there was a second silicon oxide layer with an array of "well" structures, the change of the fluorescence signal intensity with the thickness of the second silicon oxide layer was simulated. The simulation calculation results are shown in FIG. 17C, showing that when the thickness of the second silicon oxide layer was 0, that is, no second silicon oxide layer was provided, the fluorescence signal intensity was strongest, and the reflectivity decreased and the fluorescence signal intensity weakened gradually with the increase of thickness of the second silicon oxide layer. Therefore, in order to provide the "well" structures to load DNB samples, the thickness of the second silicon oxide layer was most preperably about 50 nm, and in this case, the thickness of the first silicon oxide layer was about 90 nm, and the thickness of the transition metal oxide layer was about 10 to 20 nm.

### Example 4: a method for preparing a back-illuminated sequencing chip main body with transition metal oxide "spot" structures on a quartz wafer

As shown in FIGS. 18 to 22, this example provided sectional views of processes for steps of a method for preparing a back-illuminated sequencing chip main body with transition metal oxide "spot" structures on a quartz wafer. The difference between this example and Examples 1 to 3 was that a quartz wafer or any other suitable transparent glass wafer was used as the substrate in the method of this example, and a patterned transition metal oxide layer of "spot" structures was prepared on the substrate wafer, then the chip was assembled with the patterned layer facing downward during the assembling process, and DNBs were excited by an excitation light source from the back of the chip (i.e., through the quartz wafer substrate) and the fluorescence signal was collected by the camera.

FIG. 18 shows a sectional view of a wafer structure 4-10 in which a patterned transition metal oxide layer was formed on a quartz wafer 411 with a silicon oxide layer 412, wherein the wafer was a quartz wafer but any other suitable transparent glass wafer may be used in the present disclosure. The formation processes of the oxide layer 412 and the patterned transition metal oxide layer 413 were similar to those in FIG. 1 of Example 1.

FIG. 19 shows a sectional view of multiple single chips 4-20 separated by a cutting slot formed by cutting the wafer 4-10 shown in FIG. 18. The cutting process was similar to that in FIG. 2 of Example 1.

FIG. 20 shows a sectional view of a sequencing chip 4-30 formed by packaging the single chip as shown in FIG. 19. In this process step, the sequencing chip, with the patterned layer facing downward, was assembled with and attached to a frame 431 with a liquid inflow-outflow opening 432, forming a fluid channel 433 between the frame and the patterned layer on the chip. Wherein, the frame 431 could be made of any suitable material by any suitable processing method, and the chip and the frame could be attached together with any suitable adhesive. It should be noted that this figure schematically depicted the structure that the frame should have, but this figure was not limiting, and any frame structure that could provide the function of supporting the chip, had a liquid inflow-outflow opening, and could form a fluid channel with the patterned layer of the chip should be considered within the scope of the present disclosure.

FIG. 21 shows a sectional view of a sequencing chip 4-40 formed after surface functional modification of the sequencing chip 4-30 as shown in FIG. 20. The steps of functional modification were similar to those as shown in FIG. 4 of Example 1.

FIG. 22A shows a sectional view of a sequencing chip 4-50A with a DNB array formed after loading of DNBs on the sequencing chip 4-40 that had been functionally modified as shown in FIG. 21. The DNB loading steps were similar to those in FIG. 5A of Example 1. FIG. 22A also shows the excitation light source and camera 452, which irradiated DNBs from the back of the quartz or glass substrate of the sequencing chip and collected the fluorescent signal emitted by DNBs labeled with fluorescent markers, so as to sequence the bases on DNB.

FIG. 22B shows another more concise DNB loading method that does not require surface functional modification, which was similar to that in FIG. 5B of Example 1. The excitation light source and camera 452 irradiated DNBs from the back of the quartz or glass substrate of the sequencing chip, and collected the fluorescent signal emitted by DNBs labeled with fluorescent markers, so as to sequence the bases on DNB.

In this Example 4, the silicon oxide layer was first formed on a transparent quartz wafer, then an array of transition metal oxide "spot" structures was formed on the silicon oxide layer, and the DNB sample was loaded on the transition metal oxide "spot" structure. However, in this example, the camera was placed on the back of the quartz substrate, and the light signal emitted from DNB needed to pass through the transition metal oxide layer, silicon oxide layer, and quartz substrate before being captured by the camera. Therefore, in this Example 4, the comparison of intensities of signals that can be captured by the camera after the fluorescence signal emitted by DNB passed through the transition metal oxide layer, silicon oxide layer, and quartz substrate of different thicknesses was calculated. Simulation results are shown in FIG 22C, showing that when the thickness of the silicon oxide layer was 90 nm and the thickness of the transition metal oxide layer was 10 to 20 nm (the thickness of the second silicon oxide layer was 0), the transmissivity of fluorescence emitted by DNB samples was maximum, and in this case, the intensity of the fluorescence signal passing through the transition metal oxide layer, silicon oxide layer, and quartz substrate was maximized, close to 100%.

### Example 5: a method for preparing a back-illuminated sequencing chip main body having transition metal oxide "well" structures on a quartz wafer

As shown in FIGS. 23 to 28, this Example provided sectional viewS of processes of steps of a method for preparing a back-illuminated sequencing chip main body having transition metal oxide "well" structures on a quartz wafer. A quartz wafer or any other suitable transparent glass wafer was used as the substrate in the method of this example, and a transition metal oxide layer patterned with "well" structures was prepared on the substrate wafer, then a chip was assembled with the patterned layer facing downward during the assembling process, and DNBs were excited by an excitation light source from the back of the chip (i.e., through the quartz wafer substrate) and the fluorescence signal was collected by the camera.

FIG. 23 shows a sectional view of a wafer structure 5-10 formed after a silicon oxide layer and a transition metal oxide layer were formed on the raw wafer. The formation method was similar to that in FIG. 6 of Example 2.

FIG. 24 shows a sectional view of a wafer 5-20 after the silicon oxide layer patterned with "well" structures was formed the on wafer 5-10 with the transition metal oxide layer shown in FIG. 23. The formation method thereof was similar to that in FIG. 7 of Example 2.

FIG. 25 shows a sectional view of multiple single chips 5-30 formed by cutting the wafer structure 5-20 shown in FIG. 24. The cutting process similar to that in FIG. 2 of Example 1 was used in this figure.

FIG. 26 shows a sectional view of a sequencing chip 5-30 formed by packaging the single chip shown in FIG. 25. In this process step, the sequencing chip was assembled and attached together with a frame 431 having a liquid inflow-outflow opening 432 with the patterned layer of the sequencing chipe facing downward, forming a fluid channel 433 between the frame and the patterned layer on the chip. The process in FIG. 20 of Example 3 was used in this figure.

FIG. 27 shows a sectional view of a sequencing chip 5-50 formed after surface functional modification of the sequencing chip 5-40 shown in FIG. 26. The steps of functional modification were similar to those in FIG. 4 of example 1.

FIG. 28A shows a sectional view of a sequencing chip 5-60A with a DNB array formed after loading of DNBs on the functionally modified sequencing chip 5-50 shown in FIG. 27. The DNB loading steps were similar to those in FIG. 5A of example 1. This figure also shows the excitation light source and camera 562, which irradiated DNBs from the back of the quartz or glass substrate of the sequencing chip, and collected the fluorescence signal emitted by DNBs labeled with fluorescent markers, so as to sequence the bases on DNB.

FIG. 28B shows another more concise DNB loading method that does not require surface functional modification, which was similar to that in FIG. 5B of example 1. The excitation light source and camera 562 irradiated DNBs from the back of the quartz or glass substrate of the sequencing chip, and collected the fluorescence signal emitted by DNBs labeled with fluorescent markers, so as to sequence the bases on DNB.

In this example 5, a first silicon oxide layer was formed on the quartz wafer firstly, then a transition metal oxide layer was formed on the first silicon oxide layer, and then a second silicon oxide layer with an array of "well" structures was formed on the transition metal oxide layer. In this case, the DNB sample was also loaded on the transition metal oxide layer in the "well" structures. The light signal emitted from the DNB sample was transmitted through the transition metal oxide layer, the first silicon oxide layer, and the quartz substrate, and was captured by a camera set on the back of the quartz substrate. In this case, provided that the thickness of the first silicon oxide layer was 90 nm and the thickness of the transition metal oxide layer was 10 nm or 20 nm, the influence of different thicknesses of the second silicon oxide layer on the intensity of fluorescence signal transmitted through the film layers was simulated. Simulation results are shown in FIG. 28C, showing that when the thicknesses of the first and second silicon oxide layers were the same, the fluorescence signal intensity of the structure with a transition metal oxide layer of a thickness of 10 nm was higher than that of the structure with a transition metal oxide layer of a thickness of 20 nm. And when the thickness of the first silicon oxide layer and the thickness of the transition metal oxide layer were fixed, the intensity of fluorescence signal transmitted through the film layers did not increase or decrease monotonically with the increase of the thickness of the second silicon oxide layer but showed different fluorescence signal intensity change trends under different wavelengths. In this case, when the thickness of the second silicon oxide layer ranged from 100 nm to 200 nm, it ensured that the "well" structure had an appropriate depth to load DNB and enabled the camera to collect fluorescence signals of a relatively high intensity.

### Example 6: Another method for preparing a back-illuminated sequencing chip main body having transition metal oxide "well" structures on a quartz wafer

As shown in FIGS. 29 to 34, this example provided sectional views of processes of steps of a method for preparing a back-illuminated sequencing chip main body having transition metal oxide "well" structures on a quartz wafer. The differences between this method and the method in Example 5 were that the patterned transition metal oxide layer 613 was formed on the wafer 611 containing a silicon oxide layer 612 firstly, while the transition metal oxide layer was formed on the whole wafer in the method of example 5, as shown in FIG. 29 and FIG. 23 for comparison.

FIG. 29 shows a sectional view of a wafer structure 6-10 with a patterned transition metal oxide layer 613. The formation process thereof was similar to that in FIG. 1 above.

FIG. 30 shows a sectional view of a wafer structure 6-20 formed after a silicon oxide layer 621 patterned with "well" structures 622 was formed on the wafer 6-10 with a patterned transition metal oxide layer shown in FIG. 29. The formation process thereof was similar to that in FIG. 7 of example 2, in which the "well" structures 622 on the silicon oxide layer corresponded to the patterned "spots" of the transition metal oxide layer 613 one to one, and eventually, the silicon oxide layer was higher than the transition metal oxide layer and formed an array of patterned "well" structures on the surface of the wafer. The bottom of the "well" structure was the exposed transition metal oxide layer 613.

FIG. 31 shows a sectional view of multiple single chips 6-30 separated by a cutting slot 631 formed by cutting the wafer structure 6-20 shown in FIG. 30. The cutting process was similar to that in FIG. 8 of Example 2.

FIG. 32 shows a sectional view of a sequencing chip 6-40 formed by packaging the single chip shown in FIG. 31. The process in this figure was similar to that in FIG. 26 of Example 5.

FIG. 33 shows a sectional view of a sequencing chip 6-50 formed after surface functional modification of the sequencing chip 6-40 shown in FIG. 32. The steps of functional modification were similar to those shown in FIG. 4 in Example 1.

FIG. 34A shows a sectional view of a sequencing chip 6-60A with a DNB array formed after loading of DNBs on the functionally modified sequencing chip 6-50 shown in FIG. 33. The DNB loading steps were similar to those shown in FIG. 5A of Example 1. This figure also shows the excitation light source and camera 662, which irradiated DNBs from the back of the quartz or glass substrate of the sequencing chip, and collected the fluorescent signal emitted by DNBs labeled with fluorescent markers, so as to sequence the bases on DNB.

FIG. 34B shows another more concise DNB loading method that does not require surface functional modification, similar to that shown in FIG. 5B of Example 1. The excitation light source and camera 662 irradiated DNBs from the back of the quartz or glass substrate of the sequencing chip, and collected the fluorescent signal emitted by DNBs labeled with fluorescent markers, so as to sequence the bases on DNB.

In this example 6, a first silicon oxide layer was formed on the quartz wafer firstly, then a transition metal oxide layer structured in an array of "spots" was formed on the first silicon oxide layer, and then a second silicon oxide layer with an array of "well" structures was formed obove the transition metal oxide layer. The "well" structures of the second silicon oxide layer corresponded to the "spots" of the transition metal oxide layer, and the transition metal oxide "spot" was at the bottom of the "well" structure of the second silicon oxide layer. In this case, the DNB sample was also loaded on the transition metal oxide layer in the "well" structure. The light signal emitted by the DNB sample was transmitted through the transition metal oxide layer, the first silicon oxide layer, and the quartz substrate, and was captured by a camera set on the back of the quartz substrate.

In this case, provided that the thickness of the first silicon oxide layer was 90 nm and the thickness of the transition metal oxide layer was 10 nm or 20 nm, the influence of different thicknesses of the second silicon oxide layer on the fluorescence signal intensity was simulated. Simulation results are shown in FIG. 34C, showing that when the thicknesses of the first and second silicon oxide layers were the same, the fluorescence signal intensity of the structure with a transition metal oxide layer of a thickness of 10 nm was higher than that of the structure with a transition metal oxide layer of a thickness of 20 nm. And when the thickness of the first silicon oxide layer and the thickness of the transition metal oxide layer were fixed, the intensity of the fluorescence signal transmitted through the film layers did not increase or decrease monotonically with the increase of the thickness of the second silicon oxide layer either but showed different fluorescence signal intensity change trends under different wavelengths. In this case, when the thickness of the second silicon oxide layer ranged from 100 nm to 200 nm, it ensured that the "well" structure had an appropriate depth to load DNB and enabled the camera to collect fluorescence signals of a relatively high intensity.

### Example 7: a method for preparing a sequencing chip main body with transition metal oxide "spot" structures or "well" structures on a CMOS wafer

As shown in FIGS. 35 to 41, this example provided a method for preparing a sequencing chip main body with transition metal oxide "spot" structures or "well" structures on a CMOS wafer. The difference between this method and the above methods was that, in the above methods, external excitation light and camera equipment were used, and DNBs labeled with fluorescent markers were irradiated by the excitation light with specific wavelength and energy emitted from the external excitation light source, to enable the DNBs to emit light with a specific wavelength and energy, and the light signals emitted by the DNBs were collected by the camera for sequencing, while external excitation light source and camera equipment were not required in this method. The CMOS wafer used in this method was the CMOS wafer having the function of an image sensor, each wafer may contain hundreds or thousands of image sensor chips, and each image sensor chip may have millions to tens of millions of pixels (and photodiode arrays). The light signals with different intensities from the external environment could be perceived by the image sensor chips and converted into corresponding electrical signals. The DNBs labeled with fluorescent markers were selectively loaded in the photodiode arrays on the image sensor chips, forming DNB arrays corresponding to the photodiode arrays one to one, and biological or chemical methods were used to enble DNBs to emit light (without external excitation light source). DNB arrays loaded on the image sensor chips could be sequenced by identifying light signal values at different pixels of the image sensor chip at different time.

FIG. 35 was a schematic diagram of a section of a CMOS image sensor wafer 7-10. Those skilled in the art should realize that a CMOS wafer may have multiple chips. Only two chips 71 and 72 are schematically shown in this figure. As shown in FIG. 35, the CMOS image sensor wafer had a photosensitive layer 73, an interconnection layer 74, a substrate layer 75, and a dielectric thin film layer 717 on the photosensitive layer 73. The material of such layers was usually made by stacking hafnium dioxide and tantalum pentoxide films, and a silicon oxide layer 718 was on the dielectric thin film layer 717. Wherein, the photosensitive layer 73 included a photosensitive part 716 formed in the semiconductor material 715, and the photosensitive part 716 can be a photodiode. The semiconductor material layer 715 could be made of any suitable material, such as silicon, materials of Groups III to V on silicon, graphene on silicon, silicon on insulator, and combinations thereof. Although the present disclosure described the photodiode 716, it should be noted that any suitable photosensitive structure can be applied to the present disclosure. The photosensitive diode 716 could convert the measured light signal into an electric current signal. The photosensitive diode 716 may contain source and drain of a MOS (Metal Oxide Semiconductor) transistor, which could transfer current to other components, such as to another MOS transistor. Other components may include a reset transistor, a current source follower, or a row selector for converting current values into digital signals. A dielectric layer may also be included in CMOS image sensor 10, and it was noted that this dielectric layer may contain any suitable electrical insulation material. The interconnection layer 74 contained metal wiring 714 formed in the dielectric layer 713, and the metal wiring 714 could be used for internal interconnection of integrated circuit materials as well as electrical connections to the outside. The substrate layer 75 included a silicon substrate 711 and a CMOS processing circuit layer 712, the CMOS processing circuit layer 712 may contain CMOS circuits needed for sequencing operations. For example, the CMOS processing circuit layer 712 may contain circuits for image processing, signal processing, control functions for sequencing operations, and external communications. The CMOS processing circuit 712 processed the photosensitive signal sensed by photosensitive layer 73 into an electrical signal and transmitted the electrical signal to external equipment through interconnected silicon through-hole 720 and bonding pad 719.

Those skilled in the art should be aware that the structure of the CMOS image sensor chip is described only schematically in the present disclosure, but this description is not limiting and an image sensor chip of any structure can be used in the present disclosure.

A transition metal oxide layer with an array of "spot" or "well" structures was then formed on the CMOS image sensor wafer 7-10 shown in FIG. 35, i.e., as shown in FIG. 36A, FIG. 36B, and FIG. 36C below.

FIG. 36A shows a sectional view of a CMOS wafer structure 7-20A formed after the patterned transition metal oxide layer structured in "spots" was formed on the CMOS image sensor wafer 7-10 shown in FIG. 35. The process steps in this figure were similar to those in FIG. 1 of example 1, except that the wafer in this figure was a CMOS wafer, and the transition metal oxide regions 721 of "spot" structures were distributed above the photodiode array 716.

FIG. 36B shows a sectional view of a CMOS wafer structure 7-20B formed after a patterned transition metal oxide layer with "well" structures was formed on the CMOS image sensor wafer 7-10 shown in FIG. 35. The process steps in this figure were similar to those in FIG. 6 and FIG. 7 of Example 2, except that the wafer in this figure was a CMOS wafer, and the transition metal oxide regions 724 of the "well" structures were distributed above the photodiode array 716.

FIG. 36C shows a sectional view of a CMOS wafer structure 7-20C formed after a patterned transition metal oxide layer with another "well" structures was formed on the CMOS image sensor wafer 7-10 shown in FIG. 35. The process steps in FIG. 36C were similar to those in FIG. 12 and FIG. 13 of Example 3, except that the wafer in this figure was a CMOS wafer, and the transition metal oxide regions 727 of the "well" structures were distributed above the photodiode array 716.

FIG. 37 shows a sectional view of multiple single chips 7-30 separated by a cutting slot 731 formed by cutting the CMOS wafer 7-20A with a patterned transition metal oxide layer formed in FIG. 36A (as the subsequent processes in FIG. 36A, FIG. 36B, and FIG. 36C are the same, only the patterned wafer structure shown in FIG. 36A was used to describe the subsequent inventions). The cutting process was similar to that in FIG. 2 of Example 1.

FIG. 38 shows a sectional view of a chip structure 7-40 formed after chip attachment and lead bonding of the chip shown in FIG. 37. FIG. 38 shows the first two steps in the sequencing chip assembly process. First, a single chip was attached to a package substrate 741 by glue or adhesive. The package substrate 741 could be an LGA package type substrate, the front side of the substrate had a bonding pad 742 electrically connected to the chip, the back side of the substrate had a contact 743 electrically connected to external equipment, the bonding pad 742 corresponded to the contact 743 one to one through the wiring inside the substrate. Then through the method of lead bonding, the bonding pad 719 on the chip and the bonding pad 742 on the substrate were electrically connected, so that the electrical signal transmitted from the chip was transmitted through the lead to the substrate, and then transmitted to the external equipment through the interface between the substrate and the external equipment. It should be realized that the substrate in the present disclosure includes but is not limited to the LGA form, any form of suitable packaging substrate can be applied to the present disclosure, and the glue or adhesive used in the chip attachment process also should include but is not limited to any glue or adhesive used in a packaging process, and the metal wire in wire bonding process also should include but not limited to gold wire, aluminum wire, etc.

FIG. 39 shows a sectional view of a sequencing chip 7-50 formed by attaching a lid structure to the chip structure 7-40 shown in FIG. 38. In this figure, a lid structure 751 containing a fluid channel 753, a liquid inflow-outlfow opening 752, and a supporting structure was attached to the CMOS image sensor chip and substrate by glue or adhesive to form a sequencing chip. Wherein, the fluid channel 753 was formed on top of the pattered transition metal oxide layer, and the liquid was confined within a certain space and would not contact bonding pads, leads, and other energized areas outside the fluid channel. It should be recognized that the lid 751 could be manufactured from any suitable material (including but not limited to PC, PEI, PEEK, PMMA, etc.) by any suitable processing method (including but not limited to CNC, injection molding, 3D printing, etc.). Those skilled in the art should also realize that the physical structures of the substrate 741 and the lid 751 shall include but not be limited to those shown in the figures, and any physical structure that can realize the function as shown in the figure shall be included in the present disclosure.

FIG. 40 shows a sectional view of a sequencing chip 7-60 formed after surface functional modification of the sequencing chip shown in FIG. 39. The functional modification process in this figure was similar to that in FIG. 4 of Example 1.

FIG. 41A shows a sectional view of a sequencing chip 7-70A with a DNB array formed after loading of DNBs on the functionally modified sequencing chip 7-60 shown in FIG. 40. The DNB loading process shown in this figure was similar to that in FIG. 5 of Example 1, the difference was that DNBs labeled with fluorescent markers emitted light by a biological or chemical method, without an external excitation light source, therefore the light emitted by the DNB array by the biological or chemical method could by captured by photodiode array on the image sensor and then outputted as electrical signals by the processing circuit. Based on the luminescence of the DNB array at different time and different pixels (photodiodes), the base arrangment of DNB could be identified.

FIG. 41B shows another more concise DNB loading method that does not require surface functionalization. This loading method is as shown in FIG. 5B above and the DNB luminescence signal was converted into DNB base arrangment information in the same manner as described in FIG. 41A above.

In this example 7, the transition metal oxide layer and the second silicon oxide layer formed three types of "spot" or "well" structures similar to those in the above examples on a CMOS wafer containing a photosensitive structure and a first silicon oxide layer, namely including: 1. a transition metal oxide layer structured in an array of "spots" was formed on a first silicon oxide layer, and DNB was loaded on the "spot" structure of the transition metal oxide layer; 2. a transition metal oxide film was formed on the first silicon oxide layer, a second silicon oxide layer with an array of "well" structures was formed above the transition metal oxide film, and DNB was loaded on the transition metal oxide layer at the bottom of the "well" structure of the second silicon oxide layer; 3. a transition metal oxide layer structured in an array of "spots" was formed on a first silicon oxide layer, then a second silicon oxide layer with an array of "well" structures was formed above the transition metal oxide layer, and DNB was loaded on the transition metal oxide "spot" structure at the bottom of the "well" structure of the silicon oxide layer. In these three types of "spot" or "well" structures, the light signal emitted from DNB needed to pass through the transition metal oxide, the first silicon oxide layer, the ARC (anti-reflective layer, usually tantalum pentaoxide) and PIN (usually hafnium dioxide) layers on the CMOS wafer, to be finally captured by the photosensitive structure on the CMOS wafer. Therefore, the intensity of signals that could be captured by the photosensitive structure after the light emitted by DNBs passed through these layers was simulated. The thicknesses of the PIN layer and ARC layer were determined by the process of the CMOS wafer and were usually fixed values, wherein the thickness of the PIN layer was 6 nm and the thickness of the ARC layer was 50 nm. Therefore, the influence of the thickness changes of the first silicon oxide layer, the transition metal oxide layer, and the second silicon oxide layer on the fluorescence signal intensity was simulated for the above three types of "spot" or "well" structures.

First, in the first case in this example, namely when only the first silicon oxide layer was present, the relationship between fluorescence signal intensity and the thickness of the first oxide layer was simulated. Simulation results are shown in FIG. 41C, showing that the intensity of light signals collected by the photosensitive structure in the CMOS wafer decreased monotonically with the increase of the thickness of the first oxide layer. In order to be compatible with existing standard CMOS processes and for product reliability considerations, the thickness of the oxide layer can be selected as 150 nm.

Then, provided that the thickness of the first silicon oxide layer was 150 nm, the relationship between the thickness of the transition metal oxide layer structured in an array of "spots" on the first silicon oxide layer and the fluorescence signal intensity was simulated. The simulation results are shown in FIG. 41D, showing that the intensity of light signals captured by the photosensitive structure in the CMOS wafer fluctuated with the thickness of the transition metal oxide layer. From the process point of view, the optimized thickness can be selected as 40 to 50 nm.

Then, the second case in this example 7 was simulated. provided that the thickness of the first silicon oxide layer was 150 nm, a transition metal oxide layer film was formed on the first silicon oxide layer. First, in order to determine the thickness of the transition metal oxide layer film, the relationship between the thickness and the fluorescence signal intensity was simulated. Simulation results are shown in FIG. 41E, showing that the fluorescence signal intensity fluctuated with the increase of the thickness of the transition metal oxide layer film. When the thickness was 10 to 20 nm, the fluorescence signal intensity was maximum.

Then, a second silicon oxide was formed on this basis, and the relationship between the thickness of the second silicon oxide and the fluorescence signal intensity was simulated. The simulation results are shown in FIG. 41F, showing that when the thickness of the transition metal oxide layer was fixed, the correlation between the intensity of light signals captured by this structure and the thickness of the second oxide layer was negligible. Considering the fluid demand of DNA sequencing, if the thickness of the second oxide layer was too large, the surface structure would be too deep, which could easily cause a fluid dead zone, affecting the quality of sequencing. A moderate thickness of the second oxide layer can make the DNA group to be deteced fall in the effective region more effectively, and the thickness of the second oxide layer can be selected as 50 to 100 nm.

### Example 8: Another packaging method of reusable sequencing chip

In this example, a new sequencing chip packaging method was proposed, the sequencing chip packaged by such packaging method can be reused after a special processing process, greatly reducing the cost of sequencing chips.

A patterned array of transition metal oxide "spot" or "well" structures was formed on a semiconductor wafer, and such patterned array structure may be one of the structures on the wafer as shown in FIGS. 1, 7, and 13 of examples 1 to 3. In this example, the structure in FIG. 1 of Example 1 was used as an example to describe the manufacturing process of such a reusable sequencing chip. Other sequencing chip structures included in the present disclosure can also be prepared into reusable sequencing chips by the same packaging process.

FIG. 42 shows a sectional view of a wafer structure 8-10 with a transition metal oxide layer strucuted in an array of "spots", which was same as that shown in FIG. 1 of Example 1, wherein the silicon oxide layer 812 was formed on the semiconductor wafer substrate 811 and the transition metal oxide layer 813 strucuted in "sopts" was formed on the silicon oxide layer 812. The process and material requirements of each step were consistent with the description in FIG. 1 of Example 1.

FIG. 43 shows a sectional view of a wafer structure 8-20 after multiple single chips 81 and 82 were formed by cutting the wafer structure 8-10 shown in FIG. 42, in which the cutting process was similar to that in FIG. 2 of Example 1.

FIG. 44 shows a sectional view of a reusable sequencing chip 8-30 formed by assembling the single chip 81 or 82 formed in FIG. 43 and a handle structure 831. The function of the handle structure 831 was that by fixing the handle structure with a single chip to form a sequencing chip, the handle structure can be used to capture and transfer the sequencing chip, so as to carry out DNB loading and sequencing. Only one "L"-shaped handle structure is schematically shown in FIG. 44. Those skilled in this field should realize that any handle structure that can realize the above functions is included in the present disclosure. There is no limit to the number of handle structures in the present disclosure, and multiple handle structures can be packaged with a single chip. The material of the handle structure can be plastics or metals that are compatible with DNB loading and sequencing reagents, low-cost, easy to be processed, anti-aging and anti-wear, such as polymer plastics including but not limited to polyether ether ketone, polycarbonate, polymethyl methacrylate, or metals such as aluminum alloy and stainless steel. An solid or liquid adhesive could be selected to bind the single chip to the handle structure. Any adhesives compatible with DNB loading and sequencing reagents can be used in this invention.

As shown in FIG. 45, the assembled sequencing chip shown in FIG.44 was immersed in a container 841 containing a reagent 842, wherein the reagent 842 may be any reagent used for chip surface modification, DNB loading and sequencing. During a one-time process of surface modification, DNB loading and sequencing, there may be multiple containers, respectively filled with different reagents 842. By grasping the handle structure of the sequencing chip, the sequencing chip can be switched between different containers and reagents, so as to carry out different reactions. As shown in FIG. 45, after DNBs were loaded on the DNB binding site regions ("spot" structures of the transition metal oxide layer) on the sequencing chip, light signals with different wavelengths and energies emitted by DNBs can be captured by an excitation light source and camera 843, so as to conduct sequencing operations.

After a complete sequencing, the sequencing chip with the package structure could be treated and reused. Specific treatment methods were as follows:

The sequencing chip after a complete sequencing was pre-treated and the handle structure was removed, leaving the whole chip completely exposed. The chip was then immersed in SC1 washing liquor (Slide Clean 1, a 50 mM potassium hydroxide solution containing Triton) for 10 minutes and moved out. The chip surface was repeatedly cleaned with deionized water more than 3 times, and the chip was completely dried in a nitrogen gas flow.

The SC1 washing liquor mentioned above may be replaced by SC2 washing liquor. The specific operation steps were as follows: the handle structure was removed from the sequencing chip after sequencing, then the chip was placed in SC2 washing liquor (Slide Clean 2, by mixing ammonia water and hydrogen peroxide in a certain proportion). The washing liquor was heated to 80 degrees for 5 minutes, and then the chip was taken out, repeatedly cleaned with deionized water more than 3 times, and then completely dried in a nitrogen gas flow.

The above washing liquor cleaning method could be replaced by a plasma drying treatment method. The sequencing chip after sequencing was placed in an argon plasma atmosphere for 30 minutes, then taken out, cleaned with deionized water to remove dust, and completely dried in a nitrogen gas flow.

### Example 9: Formation of microarray by changing loading conditions without modifying chip surface

The non-binding site regions were simulated by the silicon dioxide surface, and the binding site regions were simulated by the transition metal oxide surfaces of titanium dioxide and tantalum pentoxide. All three surfaces were cleaned by using a plasma cleaner, followed by further cleaning with ethanol. DNB solution with optimized conditions (changing pH of the solution and the surfactant content) (160 BP, 10 ng/uL) was used for loading DNBd on the surface of the chip. After DNB loading, cy3 dye was used for fluorescence labeling of DNBs, and then a fluorescence microscope was used for chip surface analysis. The results are shown in FIG. 46, where the bright spot is the loaded DNB, the black line is the area with relatively concentrated non-functional regions, and the density of the functional regions was relatively low (not adsorbing DNB). The sequencing chip was made from such chip according to the above assembly method, and the sequencing was performed on the Zebra platform, and results thereof are shown in FIG. 47. The loading success rate (GRR value) of DNB loading by using the new transition metal oxide array chip was higher than that of the chips manufactured by the existing technology.

Conclusion: due to the different surface properties between a metal oxide and silicon oxide, DNB can be selectively adsorbed on the functional regions of the chip surface by changing the pH of DNB solution and ingredients such as surfactant.

### Example 10: Validity check of DNB adsorption on silicon crystal with transition metal oxide spot array after selective functionalization

The silicon crystal chip with transition metal oxide spot array was cleaned by plasma cleaner and ethanol, then placed in 10mM aminoethylphosphonic acid solution, soaked for 24 hours, and then taken out, and the surface thereof was cleaned with ethanol and water. X-ray photoelectron spectroscopy (XPS) was used for elemental analysis of the three surfaces. The results shows that there was no phosphorus element on the surface of silicon oxide before and after amination, while the phosphorus element atomic concentration on the surfaces of titanium dioxide and tantalum pentoxide increased from 0 (before amination) to 2 %. DNB solution consistent with sequencing (160 BP, 10 ng/uL) was used for DNB loading on the chip surface. After DNB loading, cy3 dye was used for fluorescent labeling of DNB, and then a fluorescence microscope was used for chip surface analysis. The results are shown in FIG 48. FIG. 48 shows that the transition metal oxide modified by aminophosphonic acid had a good adsorption effect on DNB, while the silicon oxide non-binding regions (black line in the figure) had very limited adsorption for DNB.

Wherein, the silicon crystal chip with transition metal oxide spot array was prepared by oxidizing the surface of silicon dioxide wafer used in the factory and then deposting the transition metal oxide spot array by ALD.

Conclusion: there was no aminophosphonic acid on the surface of silicon dioxide, but aminophosphonic acid can be detected on the surfaces of titanium dioxide and tantalum pentoxide, which can prove the selectivity of phosphonic acid reaction. The modified surface can selectively aminate the transition metal oxide regions and achieve the specific adsorption effect of the functional regions of the chip surface for DNBs.

### Example 11: Validity check of further modification of non-functional regions by using copolymer containing polyethylene glycol

In this example, a specially customized chip was used, a transition metal oxide region on the chip had a size of 200 microns, and the interval was 500 microns. The chip was cleaned and aminophosphonic acid-modified in the same way as in Example 9, and then immersed in a 10 mg/mL polyethyleneimine-polyethylene glycol (PEI-PEG) copolymer aquesous solution for 10 minutes, followed by pure water cleaning. DNB solution consistent with sequencing (160 BP, 10 ng/uL) was then used for DNB loading on the surface of the chip. After DNB loading, cy3 dye was used for fluorescence labeling of DNBs, and then a fluorescence microscope was used for chip surface analysis. The results are shown in FIG 49. As can be seen in FIG. 49, the non-specific adsorption on the surface was further reduced after the silicon oxide non-binding region was treated with the copolymer.

Conclusion: using copolymer-containing polyethylene glycol can further reduce the adsorption of DNB and impurities on the non-functional regions of the chip surface.

### Example 12: Validity check of further modification of non-functional regions by using silane coupling agent containing polyethylene glycol

The silicon crystal chip with transition metal oxide spot array was cleaned by a plasma cleaner and ethanol, then placed in an modification solution of a silane coupling agent containing alendronic acid and polyethylene glycol for a period of reaction, then taken out and cleaned with ethanol and water. DNB solution consistent with sequencing (160 BP, 10 ng/uL) was then used for DNB loading on the chip surface. After DNB loading, cy3 dye was used for fluorescence labeling of DNBs, and then a fluorescence microscope was used for chip surface analysis. The results are shown in FIG. 50.

Conclusion: use of the silane coupling agent containing polyethylene glycol can further reduce the adsorption of the non-functional regions of the silicon oxide surface for DNBs and impurities.

### Example 13: Method for fixing DNA nano balls on chip surface using polyphosphonic acid polymer

Firstly, DNA nano balls were assembled on the transition metal oxide array chip according to the method in Examples 1 to 9, and then a 2 mg/mL solution of sodium poly(vinylphosphonate) (Mw 200,000) in PBS was injected. After standing for 10 min, the excess sodium poly(vinylphosphonate) was washed away by injecting PBS buffer. So far, fixation of DNA nano balls on the chip was completed, and then DNA sequencing was realized by hybridization of primers and adding fluorescent dNTPs. FIG. 51 is a schematic diagram of principles for fixing DNA nano balls by using the polymer in this example. DNA nano balls were assembled on the surface of a transition metal oxide chip according to a conventional method firstly, and the DNA nano balls were then fixed on the surface of the chip by the chemical reaction of phosphonic acid of sodium poly(vinylphosphonate) with the transition metal. FIG. 52 shows the stability of polymer-fixed DNA nano balls during sequencing. It can be seen that DNA nano balls were not likely to be washed out during the sequencing process and had higher stability.

### Example 14. Method for fixing DNA nano balls on chip surface using conventional protein membrane

Firstly, DNA nano balls were assembled on the transition metal oxide array chip according to the methods of examples 1 to 9, and then 2 mg/mL solution of BOVINE serum protein in PBS was injected. After standing for 10 min, the excess bovine serum protein was washed away by injecting PBS buffer, and then alcohol was injected to denature bovine serum protein. PSB was then used to wash away the alcohol in the chip, and so far, the fixation of the DNA nano balls on the chip was completed. Subsequently, DNA sequencing was realized by hybridization of primers and adding fluorescent dNTPs. FIG. 53 shows the stability of DNA nano balls fixed by protein membrane during sequencing. The stability of the DNA nano balls fixed by protein membrane was poor in sequencing.

In the present disclosure, unless expressly stated or limited otherwise, the first feature "on" or "under" the second feature may mean that the first feature directly contacts the second feature, or the first and second features may indirectly contact each other through intervening media. Also, a first feature "on", "above", and "over" a second feature may mean that the first feature is directly or obliquely above the second feature, or simply mean that the first feature is at a higher level than the second feature. A first feature "under", "below", and "at the bottom of " the second feature may mean that the first feature is directly or obliquely under the second feature, or may simply mean that the first feature is at a lower level than the second feature.

In the description of the specification, the description with reference to "one embodiment," "some embodiments," "an example," "a specific example," or "some examples" or the like means that a particular feature, structure, material, or characteristic described in connection with the embodiment or example is included in at least one embodiment or example of the present disclosure. In this specification, the schematic representations of the terms used above are not necessarily intended to refer to the same embodiment or example. Furthermore, the particular features, structures, materials, or characteristics described may be combined in any suitable manner in any one or more embodiments or examples. Moreover, various embodiments or examples and features of various embodiments or examples described in this specification can be combined and integrated by one skilled in the art without mutual contradiction.

Although embodiments of the present disclosure have been shown and described above, it will be understood that the above embodiments are exemplary and not to be construed as limiting the present disclosure and that changes, modifications, substitutions and alterations can be made to the above embodiments by those of ordinary skill in the art within the scope of the present disclosure.

## Claims

1. A sequencing chip, comprising:
a chip main body comprising a plurality of chip particles arranged in a same layer, wherein the plurality of chip particles is obtained by cutting a chip matrix along cutting lines of a wafer layer, the chip matrix comprising:
the wafer layer having the cutting lines uniformly distributed thereon;
a first silicon oxide layer made of silicon oxide and formed on an upper surface of the wafer layer; and
a transition metal oxide layer made of a transition metal oxide and formed on an upper surface of the first silicon oxide layer, and
nucleic acids fixed on the transition metal oxide layer; and
a phosphonic acid polymer film made of a polyphosphonic acid polymer and formed on an upper surface of the transition metal oxide layer.

2. The sequencing chip according to claim 1, wherein the polyphosphonic acid polymer is a poly(alkenylphosphonate) or a poly(alkenylphosphonic acid) block copolymer salt.

3. The sequencing chip according to claim 1, wherein the polyphosphonic acid polymer is:
a poly(vinylphosphonate) having a structure represented by Formula (I): 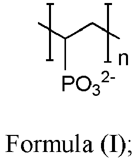 or
a poly(vinylphosphonic acid)-poly(propenylcarboxylic acid) block copolymer salt having a structure represented by Formula (II): 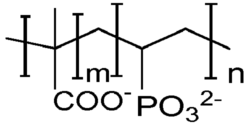 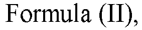 where n is an integer from 0 to 76, and m is an integer from 380 to 22,000.

4. The sequencing chip according to claim 1, wherein the nucleic acids are DNA nano balls (DNBs).

5. The sequencing chip according to any one of claims 1 to 4, wherein the polyphosphonic acid polymer is sodium poly(vinylphosphonate) or a sodium salt of a poly(vinylphosphonic acid)-poly(propenylcarboxylic acid) block copolymer.

6. The sequencing chip according to claim 1, wherein the polyphosphonic acid polymer is bonded to at least a part of transition metal oxide molecules in the transition metal oxide layer through chemical bonds;
optionally, the chemical bonds are formed by bonding the part of transition metal oxide molecules to phosphonic acid groups of the polyphosphonic acid polymer.

7. The sequencing chip according to claim 1, wherein the transition metal oxide layer is composed of a plurality of transition metal oxide spots that are not connected to each other.

8. The sequencing chip according to claim 7, wherein:
each of the plurality of transition metal oxide spots has a thickness ranging from 10 nm to 20 nm; and
the first silicon oxide layer has a thickness ranging from 80 nm to 100 nm, preferably 90 nm.

9. The sequencing chip according to claim 7, wherein:
the plurality of transition metal oxide spots further have amino groups connected thereon;
optionally, the first silicon oxide layer between the plurality of transition metal oxide spots that are not connected further has polyethylene glycol connected thereon.

10. The sequencing chip according to any one of claims 1 to 6, wherein:
the chip matrix further comprises a second silicon oxide layer;
optionally, the transition metal oxide layer is a continuous layer structure, and the second silicon oxide layer is formed by silicon oxide as a plurality of wells that are connected to each other on the upper surface of the transition metal oxide layer and a lower surface of the phosphonic acid polymer film;
optionally, the transition metal oxide layer is composed of a plurality of transition metal oxide spots that are not connected to each other, and the second silicon oxide layer is formed on the upper surface of the first silicon oxide layer and the lower surface of the phosphonic acid polymer film between the plurality of transition metal oxide spots that are not connected to each other.

11. The sequencing chip according to claim 10, wherein:
the wafer is a silicon wafer, the second silicon oxide layer has a thickness ranging from 40 nm to 60 nm, preferably 50 nm, the transition metal oxide layer has a thickness ranging from 5 nm to 15 nm, and the first silicon oxide layer has a thickness ranging from 80 nm to 100 nm, preferably 90 nm;
optionally, the wafer is a quartz wafer, the thickness of the second silicon oxide layer ranges from 100 nm to 200 nm, the thickness of the transition metal oxide layer ranges from 10 nm to 20 nm, and the thickness of the first silicon oxide layer ranges from 80 nm to 100 nm, preferably 90 nm.

12. The sequencing chip according to claim 10, wherein the transition metal oxide layer at recessed portions of the plurality of wells of the second silicon oxide layer or the plurality of transition metal oxide spots further has amino groups connected thereon;
optionally, the second silicon oxide layer further has polyethylene glycol connected thereon.

13. The sequencing chip according to claim 9 or 12, wherein:
the amino groups are bonded to at least a part of transition metal oxide molecules in the transition metal oxide layer through chemical bonds;
optionally, the chemical bonds are formed by bonding the part of transition metal oxide molecules to phosphonic acid groups of an aminophosphonic acid compound.

14. The sequencing chip according to claim 9 or 12, wherein the polyethylene glycol is provided by at least one of polyvinylenimine-polyethylene glycol and a silane coupling agent containing polyethylene glycol.

15. The sequencing chip according to claim 14, wherein the polyethylene glycol is provided by polyvinylenimine-polyethylene glycol electrostatically adsorbed on a surface of the first silicon oxide layer or a surface of the second silicon oxide layer.

16. The sequencing chip according to claim 14, wherein the polyethylene glycol is provided by a silane coupling agent containing polyethylene glycol, the sialne coupling agent being connected to the first silicon oxide layer or the second silicon oxide layer through a -Si-O-Si- chain.

17. The sequencing chip according to any one of claims 1 to 16, wherein the wafer comprises at least one selected from the group consisting of a silicon wafer, a quartz wafer, a glass wafer, and a CMOS wafer.

18. The sequencing chip according to any one of claims 1 to 16, wherein the transition metal oxide comprises at least one selected form the group consisting of titanium dioxide, zirconium dioxide, tantalum pentoxide, niobium hexoxide, and hafnium dioxide.

19. The sequencing chip according to claim 18, wherein the transition metal oxide comprises at least one selected from the group consisting of titanium dioxide, zirconia dioxide, and tantalum pentoxide.

20. A method for preparing the sequencing chip according to any one of claims 1 to 19, comprising:
1) performing surface modification on the wafer layer to obtain the chip matrix, wherein the surface modification comprises: processing a surface of the wafer layer with a transition metal oxide to form the transition metal oxide layer, the wafer layer has a first silicon oxide layer made of silicon oxide on an upper surface thereof, the transition metal layer being formed on the upper surface of the first silicon oxide layer, and the wafer layer having the cutting lines uniformly distributed thereon;
2) cutting the chip matrix along the cutting lines of the wafer layer to obtain chip particles;
3) obtaining the chip main body by assembling the chip particles;
4) fixing the nucleic acids, optionally DNA nano balls (DNBs), on the chip main body; and
5) incubating the chip main body fixed with the DNA nano balls in a buffer solution of the polyphosphonic acid polymer to obtain the sequencing chip.

21. The method according to claim 20, wherein, in step 5), the buffer solution is a PBS buffer solution.

22. The method according to claim 20, wherein, in step 5), the polyphosphonic acid polymer has a molecular weight ranging from 5 w to 510 w.

23. The method according to claim 20, wherein, in step 5), a concentration of the polyphosphonic acid polymer in the buffer solution ranges from 1.5 mg/mL to 2.5 mg/mL, preferably 2 mg/mL.

24. The method according to claim 20, wherein, in step 5), said incubating is performed at a room temperature for 8 min to 12 min.

25. The method according to claim 20, wherein, in step 1), the first silicon oxide layer is formed in advance on the upper surface of the wafer layer by low-temperature plasma chemical vapor deposition, plasma-enhanced chemical vapor deposition, sputtering, or atomic layer deposition.

26. The method according to claim 20, wherein, in step 1), the surface modification on the wafer layer is performed by thin film deposition, photoetching, or etching, to form a continuous transition metal oxide layer or a metal oxide layer arranged as spots.

27. The method according to claim 26, wherein:
the transition metal oxide layer is a continuous layer structure; and in step 1), the mehtod further comprises: forming a second silicon oxide layer made of silicon oxide in a continuous arrangement of wells on the upper surface of the transition metal oxide layer;
optionally, the transition metal oxide layer is arranged as spots; and in step 1), the method further comprises: forming the second silicon oxide layer by depositing silicon oxide between the spots of the transition metal oxide layer.

28. The method according to any one of claims 20 to 27, further comprising, subsequent to step 3) and prior to step 4):
preforming amination treatment on the transition metal oxide.

29. The method according to claim 28, wherein the amination treatment is performed by reacting the transition metal oxide with an aminophosphonic acid compound.

30. The method according to any one of claims 20 to 29, further comprising, subsequent to step 3) and prior to step 4):
performing surface modification on the first silicon oxide layer or the second silicon oxide layer, to introduce polyethylene glycol into the first silicon oxide layer or the second silicon oxide layer.

31. The method according to claim 30, wherein the polyethylene glycol is provided by at least one of polyvinylenimine-polyethylene glycol and a silane coupling agent containing polyethylene glycol;
optionally, the polyethylene glycol is provided by polyvinylenimine-polyethylene glycol, and the surface modification is performed by electrostatic adsorption of the polyvinylenimine-polyethylene glycol to a surface of the first silicon oxide layer or a surface of the second silicon oxide layer;
optionally, the polyethylene glycol is provided by a silane coupling agent containing the polyethylene glycol, and the surface modification is performed by condensation reaction of the silane coupling agent containing the polyethylene glycol with hydroxyl groups of the first silicon oxide layer or the second silicon oxide layer, wherein the hydroxyl groups are provided by Si-OH formed after the first or second silicon oxide layer is ionized and then adsorbs hydroxide ions in water.

32. The method according to claim 20, wherein, in step 2), said cutting is performed by a semiconductor wafer cutting method.

33. The method according to claim 20, wherein, in step 3), said assembling comprises:
placing the chip particle in a support frame having a liquid inflow-outflow opening; and
attacting the chip particle to the support frame with a glue or an adhesive, a fluid channel being formed between the support frame and the chip particle.

34. The method according to claim 33, wherein the wafer is a silicon wafer, and said assembling comprises:
attaching the chip particle to the support frame with an upper surface of the chip particle facing upward; and
providing a cover glass on the upper surface of the chip particle to obtain the chip main body.

35. The method according to claim 33, wherein the wafer is a quartz wafer or a glass wafer, and said assembling comprises:
attaching the chip particle to the support frame with a lower surface of the chip particle facing upward to obtain the chip main body.

36. The method according to claim 20, wherein the wafer is a CMOS wafer, and said assembling comprises:
attaching a lower surface of the chip particle to a substrate; and
bonding the chip particle to the substrate through a lead wire to obtain the chip main body, the lead wire being configured to transmit an electrical signal on the chip particle to the substrate.

37. A sequencing method, comprising:
performing sequencing by using the sequencing chip according to any one of claims 1 to 19 or the sequencing chip prepared by the method according to any one of claims 20 to 36.
